(19) Europäisches Patentamt
European Patent Office
Office européen des brevets

(11) EP 3 044 286 B1

(12) # EUROPÄISCHE PATENTSCHRIFT

(45) Veröffentlichungstag und Bekanntmachung des Hinweises auf die Patenterteilung:
**31.01.2018 Patentblatt 2018/05**

(21) Anmeldenummer: **14755593.2**

(22) Anmeldetag: **13.08.2014**

(51) Int Cl.:
***C09K 11/06*** *(2006.01)* ***H01L 51/50*** *(2006.01)*

(86) Internationale Anmeldenummer:
**PCT/EP2014/002225**

(87) Internationale Veröffentlichungsnummer:
**WO 2015/036080 (19.03.2015 Gazette 2015/11)**

(54) **ORGANISCHE ELEKTROLUMINESZENZVORRICHTUNG**

ORGANIC ELECTROLUMINESCENT DEVICE

DISPOSITIF D'ÉLECTROLUMINESCENCE ORGANIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.09.2013 EP 13004412**

(43) Veröffentlichungstag der Anmeldung:
**20.07.2016 Patentblatt 2016/29**

(73) Patentinhaber: **Merck Patent GmbH**
**64293 Darmstadt (DE)**

(72) Erfinder:
- **PARHAM, Amir Hossain**
**60486 Frankfurt am Main (DE)**
- **STOESSEL, Philipp**
**60389 Frankfurt am Main (DE)**
- **PFLUMM, Christof**
**64291 Darmstadt (DE)**
- **JATSCH, Anja**
**60489 Frankfurt am Main (DE)**
- **KAISER, Joachim**
**64289 Darmstadt (DE)**

(56) Entgegenhaltungen:
**US-A1- 2009 030 202** **US-A1- 2011 260 138**
**US-A1- 2012 256 169**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Anmeldung betrifft eine organische Elektrolumineszenzvorrichtung (OLED) enthaltend eine emittierende Schicht, wobei die emittierende Schicht eine Verbindung mit einem geringen Unterschied zwischen den Energien des $S_1$- und des $T_1$-Zustands sowie zusätzlich eine weitere Verbindung einer Formel (I) oder (II) enthält.

Allgemein wird unter der Bezeichnung OLED eine elektronische Vorrichtung verstanden, welche mindestens ein organisches Material enthält und welche unter Anlegen von elektrischer Spannung Licht emittiert. Der grundsätzliche Aufbau von OLEDs ist dem Fachmann bekannt und unter anderem in US 4539507, US 5151629, EP 0676461 und WO 98/27136 beschrieben.

Die Energien des $S_1$- und des $T_1$-Zustands einer Verbindung sind im Rahmen der vorliegenden Anmeldung definiert als diejenigen Energien, die für die betreffenden Zustände der Verbindung durch quantenchemische Rechnungen erhalten werden. Der $S_1$-Zustand ist dabei der energetisch am niedrigsten liegende angeregte Singulettzustand, und der $T_1$-Zustand ist der energetisch am niedrigsten liegende Triplettzustand. Wie die quantenchemischen Rechnungen genau durchgeführt werden, ist in den Ausführungsbeispielen beschrieben.

Bei der Entwicklung von neuartigen OLEDs besteht hohes Interesse an einer Verbesserung der Effizienz und der Betriebsspannung der Vorrichtungen. Weiterhin besteht Interesse an einer Verbesserung der Lebensdauer der Vorrichtungen. Nochmals weiterhin besteht Interesse an der Bereitstellung von OLEDs, welche einfach und kostengünstig herzustellen sind, und welche inbesondere aus einfach herstellbaren und kostengünstigen Materialien aufgebaut werden können.

Im Stand der Technik ist bekannt, dass mit bestimmten rein organischen emittierenden Verbindungen, welche nicht phosphoreszieren, sondern fluoreszieren, OLEDs mit sehr guten Effizienzen erhalten werden können. Beispielsweise ist in H. Uoyama et al., Nature 2012, 492, 234, offenbart, dass mit Carbazolyl-Cyanobenzolverbindungen als emittierende Verbindungen OLEDs mit externen Quanteneffizienzen erhalten werden können, die ähnlich gut oder besser als diejenigen sind, die mit phosphoreszierenden Emittern erhalten werden können.

Die in dieser Publikation verwendeten emittierenden Verbindungen weisen einen geringen Energieunterschied zwischen $S_1$- und $T_1$-Zustand auf. Bevorzugt liegt der Energieunterschied im Bereich der thermischen Energie oder darunter. In der genannten Publikation wird die Verwendung dieser emittierenden Verbindungen in der emittierenden Schicht in Kombination mit einer weiteren Verbindung beschrieben, die ein Matrixmaterial darstellt. Als Matrixmaterial ist unter anderem das Carbazolderivat CBP und das Aryl-Phosphinoxid PPT offenbart.

Weiterhin ist im Stand der Technik bekannt, dass die oben genannten emittierenden Verbindungen mit geringem Energieunterschied zwischen $S_1$- und $T_1$-Zustand vorteilhaft auch in Kombination mit der Aryl-Silanverbindung TBSi-F als Matrixmaterial eingesetzt werden können (G. Mehes, Angew. Chem. Int. Ed. 2012, 51, 11311-11315).

US 2009/030202 und US 2011/260138 und US 2012/259169 offenbaren Dibenzofuran- und Dibenzothiophenderivate zur Verwendung in OLEDs in Kombination mit Metallkomplexen.

Trotz dieser Fortschritte besteht weiterhin Verbesserungsbedarf auf dem Gebiet der OLEDs, inbesondere auf dem Gebiet der Zusammensetzungen für die emittierende Schicht dieser Vorrichtungen. Auch besteht hohes Interesse an der Bereitstellung von alternativen Ausführungsformen für die emittierende Schicht von OLEDs, die zu Leistungsdaten der OLEDs führen, die mit denjenigen im Stand der Technik vergleichbar sind.

Hierzu wurden im Rahmen der vorliegenden Anmeldung Untersuchungen angestellt, welche Verbindungen sich als Matrixmaterialien zur Verwendung in Kombination mit den oben genannten emittierenden Verbindungen eignen. Dazu steht dem Fachmann eine unüberschaubare Vielzahl an Strukturklassen gegenüber, welche als geeignet bekannt sind, in OLEDs als Funktionsmaterialien eingesetzt zu werden.

In diesen Untersuchungen wurde nun überraschend gefunden, dass Aryl-Dibenzofuran-Verbindungen und Aryl-Dibenzothiophen-Verbindungen mit bestimmter Struktur sich hervorragend zur Verwendung als Matrixmaterialien in Kombination mit den oben genannten emittierenden Verbindungen mit geringem Energieunterschied zwischen $S_1$- und $T_1$-Zustand eignen. Dabei werden unter anderem sehr gute Werte für die Betriebsspannung U1000, die externe Quanteneffizienz EQE, die Lebensdauer und den Roll-Off (Zur Erläuterung vgl. Ausführungsbeispiele) erhalten. Im Vergleich zu Elektrolumineszenzvorrichtungen enthaltend Iridium- oder Platinkomplexe als Emitter wird bei den erfindungsgemäßen Vorrichtungen eine längere Lebensdauer bei erhöhter Temperatur erhalten.

Gegenstand der vorliegenden Anmeldung ist somit eine organische Elektrolumineszenzvorrichtung, gemäß Anspruch 1.

Z ist bei jedem Auftreten gleich oder verschieden $CR^1$, C oder N, wobei Z genau dann gleich C ist, wenn eine Gruppe $L^1$ bzw. $L^2$ zusammen mit der daran gebundenen Gruppe an Z gebunden ist;

$L^1$, $L^2$ ist bei jedem Auftreten gleich oder verschieden eine Einfachbindung oder eine divalente Gruppe;

$Ar^1$ ist bei jedem Auftreten gleich oder verschieden ein aromatisches oder heteroaromatisches Ringsystem mit 5

bis 40 aromatischen Ringatomen, das mit einem oder mehreren Resten $R^2$ substituiert sein kann;

$R^1$, $R^2$ ist bei jedem Auftreten gleich oder verschieden H, D, F, $C(=O)R^3$, CN, $Si(R^3)_3$, $N(R^3)_2$, $P(=O)(R^3)_2$, $OR^3$, $S(=O)R^3$, $S(=O)_2R^3$, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten $R^3$ substituiert sein können und wobei eine oder mehrere $CH_2$-Gruppen in den oben genannten Gruppen durch $-R^3C=CR^3-$, -C≡C-, $Si(R^3)_2$, C=O, $C=NR^3$, -C(=O)O-, $-C(=O)NR^3-$, $NR^3$, $P(=O)(R^3)$, -O-, -S-, SO oder $SO_2$ ersetzt sein können, oder ein aromatisches oder heteroaromatisches Ringsystem mit 5 bis 30 aromatischen Ringatomen, das jeweils durch einen oder mehrere Reste $R^3$ substituiert sein kann, wobei zwei oder mehr Reste $R^1$ bzw. $R^2$ miteinander verknüpft sein können und einen Ring bilden können;

$R^3$ ist bei jedem Auftreten gleich oder verschieden H, D, F, $C(=O)R^4$, CN, $Si(R^4)_3$, $N(R^4)_2$, $P(=O)(R^4)_2$, $OR^4$, $S(=O)R^4$, $S(=O)_2R^4$, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten $R^4$ substituiert sein können und wobei eine Unter einer divalenten Gruppe wird im Rahmen der vorliegenden Anmeldung eine beliebige organische Gruppe verstanden, die zwei freie Bindungen aufweist. Dies kann beispielsweise ein Heteroatom, eine Kohlenwasserstoffkette, ein Kohlenwasserstoffring, oder eine Aneinanderreihung bzw. Verbindung mehrerer der oben genannten Einheiten sein. Dabei können die Einheiten substituiert oder unsubstituiert sein.

Eine Arylgruppe im Sinne dieser Erfindung enthält 6 bis 60 aromatische Ringatome; eine Heteroarylgruppe im Sinne dieser Erfindung enthält 5 bis 60 aromatische Ringatome, von denen mindestens eines ein Heteroatom darstellt. Die Heteroatome sind bevorzugt ausgewählt aus N, O und S. Dies stellt die grundlegende Definition dar. Werden in der Beschreibung der vorliegenden Erfindung andere Bevorzugungen angegeben, beispielsweise bezüglich der Zahl der aromatischen Ringatome oder der enthaltenen Heteroatome, so gelten diese.

Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin oder Thiophen, oder ein kondensierter (annellierter) aromatischer bzw. heteroaromatischer Polycyclus, beispielsweise Naphthalin, Phenanthren, Chinolin oder Carbazol verstanden. Ein kondensierter (annellierter) aromatischer bzw. heteroaromatischer Polycyclus besteht im Sinne der vorliegenden Anmeldung aus zwei oder mehr miteinander kondensierten einfachen aromatischen bzw. heteroaromatischen Cyclen.

Unter einer Aryl- oder Heteroarylgruppe, die jeweils mit den oben genannten Resten substituiert sein kann und die über beliebige Positionen am Aromaten bzw. Heteroaromaten verknüpft sein kann, werden insbesondere Gruppen verstanden, welche abgeleitet sind von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, Pyrazin, Phenazin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ein aromatisches Ringsystem im Sinne dieser Erfindung enthält 6 bis 60 C-Atome im Ringsystem. Ein heteroaromatisches Ringsystem im Sinne dieser Erfindung enthält 5 bis 60 aromatische Ringatome, von denen mindestens eines ein Heteroatom darstellt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein $sp^3$-hybridisiertes C-, Si-, N- oder O-Atom, ein $sp^2$-hybridisiertes C- oder N-Atom oder ein sp-hybridisiertes C-Atom, verbunden sein können. So sollen beispielsweise auch Systeme wie 9,9'-Spirobifluoren, 9,9'-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkyl-, Alkenyl- oder Alkinylgruppe oder durch eine Silylgruppe verbunden sind. Weiterhin werden auch Systeme, in denen zwei oder

mehr Aryl- oder Heteroarylgruppen über Einfachbindungen miteinander verknüpft sind, als aromatische oder heteroaromatische Ringsysteme im Sinne dieser Erfindung verstanden, wie beispielsweise Systeme wie Biphenyl, Terphenyl oder Diphenyltriazin.

Unter einem aromatischen oder heteroaromatischen Ringsystem mit 5 - 60 aromatischen Ringatomen, welches noch jeweils mit Resten wie oben definiert substituiert sein kann und welches über beliebige Positionen am Aromaten bzw. Heteroaromaten verknüpft sein kann, werden insbesondere Gruppen verstanden, die abgeleitet sind von Benzol, Naphthalin, Anthracen, Benzanthracen, Phenanthren, Benzphenanthren, Pyren, Chrysen, Perylen, Fluoranthen, Naphthacen, Pentacen, Benzpyren, Biphenyl, Biphenylen, Terphenyl, Terphenylen, Quaterphenyl, Fluoren, Spirobifluoren, Dihydrophenanthren, Dihydropyren, Tetrahydropyren, cis- oder trans-Indenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol oder Kombinationen dieser Gruppen.

Im Rahmen der vorliegenden Erfindung werden unter einer geradkettigen Alkylgruppe mit 1 bis 40 C-Atomen bzw. einer verzweigten oder cyclischen Alkylgruppe mit 3 bis 40 C-Atomen bzw. einer Alkenyl- oder Alkinylgruppe mit 2 bis 40 C-Atomen, in der auch einzelne H-Atome oder $CH_2$-Gruppen durch die oben bei der Definition der Reste genannten Gruppen substituiert sein können, bevorzugt die Reste Methyl, Ethyl, n-Propyl, i-Propyl, n-Butyl, i-Butyl, s-Butyl, t-Butyl, 2-Methylbutyl, n-Pentyl, s-Pentyl, Cyclopentyl, neoPentyl, n-Hexyl, Cyclohexyl, neo-Hexyl, n-Heptyl, Cycloheptyl, n-Octyl, Cyclooctyl, 2-Ethylhexyl, Trifluormethyl, Pentafluorethyl, 2,2,2-Trifluorethyl, Ethenyl, Propenyl, Butenyl, Pentenyl, Cyclopentenyl, Hexenyl, Cyclohexenyl, Heptenyl, Cycloheptenyl, Octenyl, Cyclooctenyl, Ethinyl, Propinyl, Butinyl, Pentinyl, Hexinyl oder Octinyl verstanden. Unter einer Alkoxy- oder Thioalkylgruppe mit 1 bis 40 C-Atomen werden bevorzugt Methoxy, Trifluormethoxy, Ethoxy, n-Propoxy, i-Propoxy, n-Butoxy, i-Butoxy, s-Butoxy, t-Butoxy, n-Pentoxy, s-Pentoxy, 2-Methylbutoxy, n-Hexoxy, Cyclohexyloxy, n-Heptoxy, Cycloheptyloxy, n-Octyloxy, Cyclooctyloxy, 2-Ethylhexyloxy, Pentafluorethoxy, 2,2,2-Trifluorethoxy, Methylthio, Ethylthio, n-Propylthio, i-Propylthio, n-Butylthio, i-Butylthio, s-Butylthio, t-Butylthio, n-Pentylthio, s-Pentylthio, n-Hexylthio, Cyclohexylthio, n-Heptylthio, Cycloheptylthio, n-Octylthio, Cyclooctylthio, 2-Ethylhexylthio, Trifluormethylthio, Pentafluorethylthio, 2,2,2-Trifluorethylthio, Ethenylthio, Propenylthio, Butenylthio, Pentenylthio, Cyclopentenylthio, Hexenylthio, Cyclohexenylthio, Heptenylthio, Cycloheptenylthio, Octenylthio, Cyclooctenylthio, Ethinylthio, Propinylthio, Butinylthio, Pentinylthio, Hexinylthio, Heptinylthio oder Octinylthio verstanden.

Unter der Formulierung, dass zwei oder mehr Reste miteinander einen Ring bilden können, soll im Rahmen der vorliegenden Anmeldung unter anderem verstanden werden, dass die beiden Reste miteinander durch eine chemische Bindung verknüpft sind. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet.

Es ist bevorzugt, dass nicht mehr als zwei Gruppen Z pro Ring gleich N sind, besonders bevorzugt ist nicht mehr als eine Gruppe Z pro Ring gleich N.

Gemäß einer bevorzugten Ausführungsform ist Z bei jedem Auftreten gleich oder verschieden $CR^1$ oder C, wobei Z genau dann gleich C ist, wenn eine Gruppe $L^1$ bzw. $L^2$ zusammen mit der daran gebundenen Gruppe an Z gebunden ist.

Es ist bevorzugt, dass Y bei jedem Auftreten gleich oder verschieden O oder S ist.

Es ist bevorzugt, dass $L^1$ bei jedem Auftreten gleich oder verschieden gewählt ist aus einer Einfachbindung, $Si(R^2)_2$, O, S oder einer Alkylengruppe mit 1 bis 10 C-Atomen, bei der eine oder mehrere $CH_2$-Gruppen durch $Si(R^2)_2$, O, S, C=O, $C=NR^2$, C=O-O, $C=O-NR^2$, $NR^2$, $P(=O)(R^2)$, SO oder $SO_2$ ersetzt sein können und die mit einem oder mehreren Resten $R^2$ substituiert sein kann, oder einem aromatischen oder heteroaromatischen Ringsystem mit 5 bis 30 aromatischen Ringatomen, das jeweils durch einen oder mehrere Reste $R^2$ substituiert sein kann.

Besonders bevorzugt ist $L^1$ bei jedem Auftreten gleich oder verschieden gewählt aus einer Einfachbindung, $Si(R^2)_2$ oder einem aromatischen oder heteroaromatischen Ringsystem mit 5 bis 30 aromatischen Ringatomen, das mit einem oder mehreren Resten $R^2$ substituiert sein kann; ganz besonders bevorzugt ist $L^1$ bei jedem Auftreten gleich oder verschieden gewählt aus einer Einfachbindung oder einem aromatischen oder heteroaromatischen Ringsystem mit 5 bis 30 aromatischen Ringatomen, das mit einem oder mehreren Resten $R^2$ substituiert sein kann.

Es ist bevorzugt, dass $L^2$ gewählt ist aus einer Einfachbindung, $Si(R^2)_2$, O, S oder einer Alkylengruppe mit 1

bis 10 C-Atomen, bei der eine oder mehrere $CH_2$-Gruppen durch $Si(R^2)_2$, O, S, C=O, C=N$R^2$, C=O-O, C=O-N$R^2$, N$R^2$, P(=O)($R^2$), SO oder $SO_2$ ersetzt sein können und die mit einem oder mehreren Resten $R^2$ substituiert sein kann, oder einem aromatischen oder heteroaromatischen Ringsystem mit 5 bis 60 aromatischen Ringatomen, das jeweils durch einen oder mehrere Reste $R^2$ substituiert sein kann.

Besonders bevorzugt ist $L^2$ gewählt aus einer Einfachbindung oder einem aromatischen oder heteroaromatischen Ringsystem mit 5 bis 60 aromatischen Ringatomen, das mit einem oder mehreren Resten $R^2$ substituiert sein kann; ganz besonders bevorzugt ist $L^2$ ein aromatisches oder heteroaromatisches Ringsystem mit 5 bis 60 aromatischen Ringatomen, das mit einem oder mehreren Resten $R^2$ substituiert sein kann.

Es ist bevorzugt, dass $Ar^1$ bei jedem Auftreten gleich oder verschieden ein aromatisches oder heteroaromatisches Ringsystem mit 5 bis 18 aromatischen Ringatomen, besonders bevorzugt 5 bis 16 aromatischen Ringatomen ist, das mit einem oder mehreren Resten $R^2$ substituiert sein kann. Besonders bevorzugt ist $Ar^1$ gewählt aus wahlweise mit Resten $R^2$ substituiertem Phenyl, Biphenyl, Terphenyl, Quaterphenyl, Fluorenyl, Spirobifluorenyl, Indenofluorenyl, Naphthyl, Anthracenyl, Phenanthrenyl, Pyrenyl, Fluoranthenyl, Furanyl, Benzofuranyl, Isobenzofuranyl, Dibenzofuranyl, Thiophenyl, Benzothiophenyl, Isobenzothiophenyl, Dibenzothiophenyl, Pyrrolyl, Indolyl, Isoindolyl, Carbazolyl, Indolocarbazolyl, Indenocarbazolyl, Pyridyl, Chinolinyl, Isochinolinyl, Acridyl, Pyrazolyl, Imidazolyl, Benzimidazolyl, Pyridazyl, Pyrimidyl, Pyrazinyl und Phenanthrolyl.

Es ist bevorzugt, dass $R^1$ bei jedem Auftreten gleich oder verschieden gewählt ist aus H, D, F, CN, $Si(R^3)_3$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, wobei die oben genannten Gruppen jeweils mit einem oder mehreren Resten $R^3$ substituiert sein können und wobei in den oben genannten Gruppen eine oder mehrere $CH_2$-Gruppen durch -C≡C-, -$R^3$C=C$R^3$-, $Si(R^3)_2$, C=O, C=N$R^3$, -N$R^3$-, -O-, -S-, -C(=O)O- oder -C(=O)N$R^3$- ersetzt sein können, oder einem aromatischen oder heteroaromatischen Ringsystem mit 5 bis 20 aromatischen Ringatomen, das jeweils mit einem oder mehreren Resten $R^3$ substituiert sein kann, wobei zwei oder mehr Reste $R^1$ miteinander verknüpft sein können und einen Ring bilden können.

Besonders bevorzugt ist $R^1$ bei jedem Auftreten gleich oder verschieden gewählt aus H, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppen jeweils mit einem oder mehreren Resten $R^3$ substituiert sein können, oder einem aromatischen oder heteroaromatischen Ringsystem mit 5 bis 20 aromatischen Ringatomen, das jeweils mit einem oder mehreren Resten $R^3$ substituiert sein kann.

Es ist bevorzugt, dass $R^2$ bei jedem Auftreten gleich oder verschieden gewählt ist aus H, D, F, CN, $Si(R^3)_3$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, wobei die oben genannten Gruppen jeweils mit einem oder mehreren Resten $R^3$ substituiert sein können und wobei in den oben genannten Gruppen eine oder mehrere $CH_2$-Gruppen durch -C≡C-, -$R^3$C=C$R^3$-, $Si(R^3)_2$, C=O, C=N$R^3$, -N$R^3$-, -O-, -S-, -C(=O)O- oder -C(=O)N$R^3$- ersetzt sein können, oder einem aromatischen oder heteroaromatischen Ringsystem mit 5 bis 20 aromatischen Ringatomen, das jeweils mit einem oder mehreren Resten $R^3$ substituiert sein kann, wobei zwei oder mehr Reste $R^2$ miteinander verknüpft sein können und einen Ring bilden können.

Besonders bevorzugt ist $R^2$ bei jedem Auftreten gleich oder verschieden gewählt aus H, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppen jeweils mit einem oder mehreren Resten $R^3$ substituiert sein können, oder einem aromatischen oder heteroaromatischen Ringsystem mit 5 bis 20 aromatischen Ringatomen, das jeweils mit einem oder mehreren Resten $R^3$ substituiert sein kann.

Es ist bevorzugt, dass $R^3$ bei jedem Auftreten gleich oder verschieden gewählt ist aus H, D, F, CN, $Si(R^4)_3$, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen, wobei die oben genannten Gruppen jeweils mit einem oder mehreren Resten $R^4$ substituiert sein können und wobei in den oben genannten Gruppen eine oder mehrere $CH_2$-Gruppen durch -C≡C-, -$R^4$C=C$R^4$-, $Si(R^4)_2$, C=O, C=N$R^4$, -N$R^4$-, -O-, -S-, -C(=O)O- oder -C(=O)N$R^4$- ersetzt sein können, oder einem aromatischen oder heteroaromatischen Ringsystem mit 5 bis 20 aromatischen Ringatomen, das jeweils mit einem oder mehreren Resten $R^4$ substituiert sein kann, wobei zwei oder mehr Reste $R^3$ miteinander verknüpft sein können und einen Ring bilden können.

Besonders bevorzugt ist $R^3$ bei jedem Auftreten gleich oder verschieden gewählt aus H, F, CN, einer geradkettigen Alkylgruppe mit 1 bis 10 C-Atomen oder einer verzweigten oder cyclischen Alkylgruppe mit 3 bis 10 C-Atomen, wobei die Alkylgruppen jeweils mit einem oder mehreren Resten $R^4$ substituiert sein können, oder einem aromatischen oder heteroaromatischen Ringsystem mit 5 bis 20 aromatischen Ringatomen, das jeweils mit einem oder mehreren Resten $R^4$ substituiert sein kann.

Es gilt allgemein und für alle bevorzugten Ausführungsformen der Formel (I), dass die Anbindungspositionen von $L^1$ in den Positionen gewählt aus Positionen 1, 2, 3, 4, 6, 7, 8 und 9 des zentralen Grundkörpers, wie unten gezeigt, vorliegen können.

Es gilt allgemein und für alle bevorzugten Ausführungsformen der Formel (II), dass die Anbindungspositionen von $L^2$ in den Positionen gewählt aus Positionen 1, 2, 3 und 4 des zentralen Grundkörpers, wie unten gezeigt, vorliegen können.

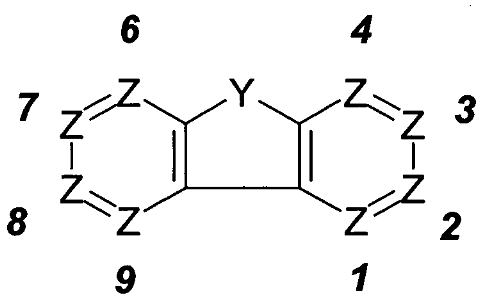

Bevorzugte Ausführungsformen der Formel (I) entsprechen den folgenden Formeln (I-1) bis (I-4)

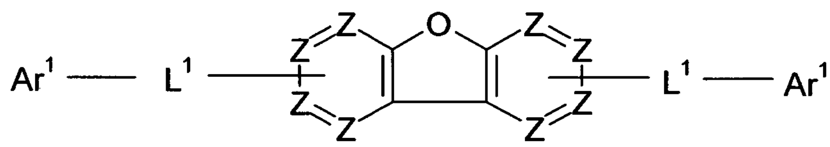

Formel (I-1)

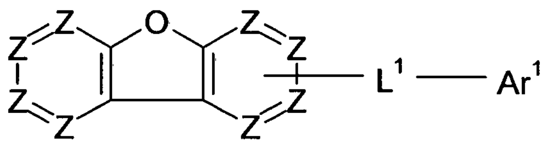

Formel (I-2)

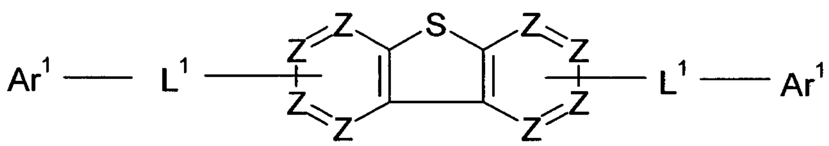

Formel (I-3)

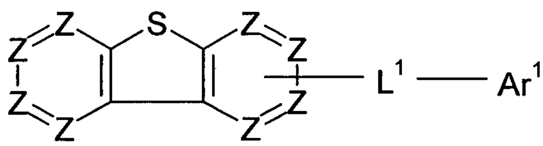

Formel (I-4),

wobei die auftretenden Gruppen definiert sind wie oben.

Bevorzugt entsprechen die auftretenden Gruppen in Formeln (I-1) bis (I-4) ihren oben angegebenen bevorzugten Ausführungsformen.

Bevorzugte Ausführungsformen von Formel (I-1) und (I-3) sind die folgenden Formeln (I-1-1) bis (I-1-2) und (I-3-1) bis (I-3-2)

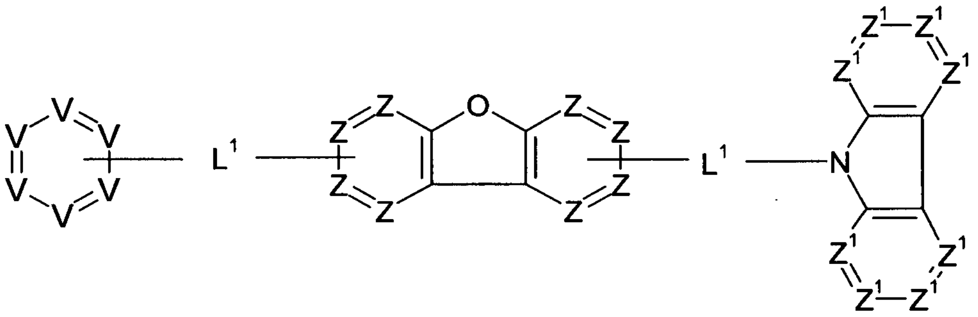

Formel (I-1-1)

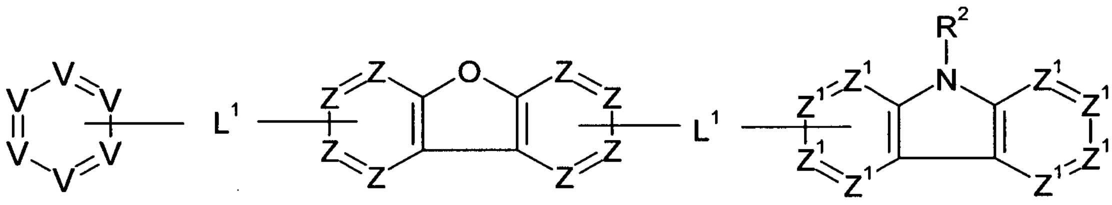

Formel (I-1-2)

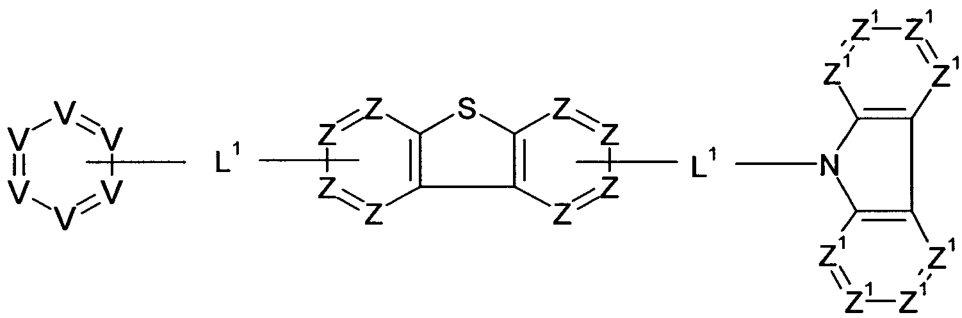

Formel (I-3-1)

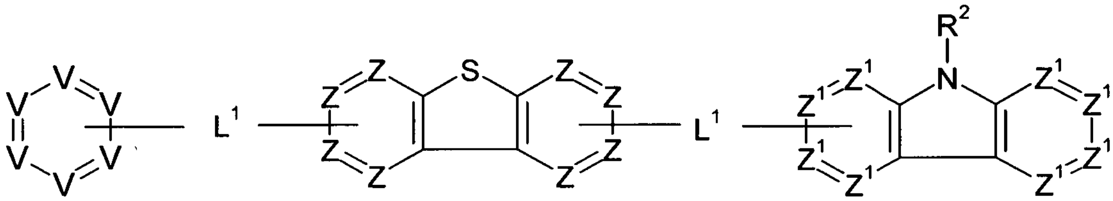

Formel (I-3-2),

wobei gilt:

V ist bei jedem Auftreten gleich oder verschieden $CR^2$, C oder N, wobei V genau dann gleich C ist, wenn eine Gruppe $L^1$ daran gebunden ist, und wobei die Maßgabe gilt, dass mindestens eine Gruppe V gleich N ist;

$Z^1$ ist bei jedem Auftreten gleich oder verschieden $CR^2$, C oder N, wobei $Z^1$ genau dann gleich C ist, wenn eine Gruppe $L^1$ daran gebunden ist;

$Ar^2$ ist eine kondensierte Arylgruppe mit 10 bis 14 aromatischen Ringatomen, die mit einem oder mehreren Resten $R^2$ substituiert sein kann;

und wobei die weiteren auftretenden Gruppen definiert sind wie oben.

Bevorzugt entsprechen die auftretenden Gruppen in Formeln (I-1-1) bis (I-1-2) und (I-3-1) bis (I-3-2) ihren oben angegebenen bevorzugten Ausführungsformen.

Weiterhin ist es bevorzugt, dass $Z^1$ gleich $CR^2$ oder C ist, wobei $Z^1$ genau dann gleich C ist, wenn eine Gruppe $L^1$ daran gebunden ist.

Weiterhin ist es bevorzugt, dass genau 1, 2 oder 3 Gruppen V im Ring gleich N sind, und die restlichen Gruppen V gleich $CR^2$ bzw. C sind. Dabei ist es bevorzugt, dass nicht mehr als zwei benachbarte Gruppen V im Ring gleich N sind, besonders bevorzugt dass keine benachbarten Gruppen V im Ring gleich N sind.

Weiterhin ist es für Formeln (I-1-1) bis (I-1-2) und (I-3-1) bis (I-3-2) bevorzugt, dass $L^1$ eine Einfachbindung oder eine wahlweise mit Resten $R^2$ substituierte Phenylgruppe oder Biphenylgruppe ist.

Bevorzugte Ausführungsformen von Formel (I-2) und (I-4) sind die folgenden Formeln (I-2-1) bis (I-2-4) und (I-4-1) bis (I-4-4)

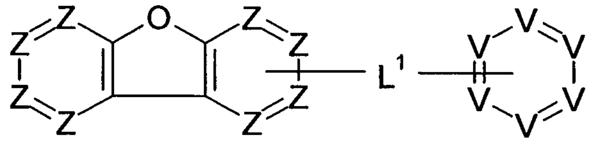

Formel (I-2-1)

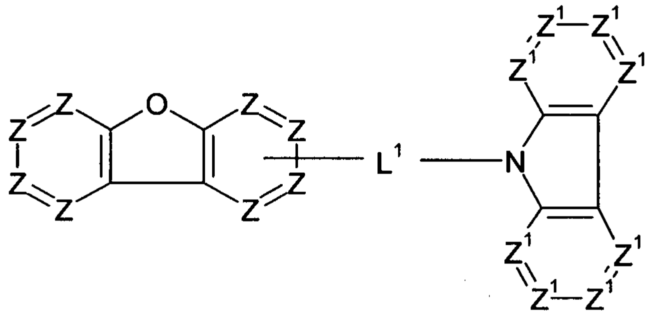

Formel (I-2-2)

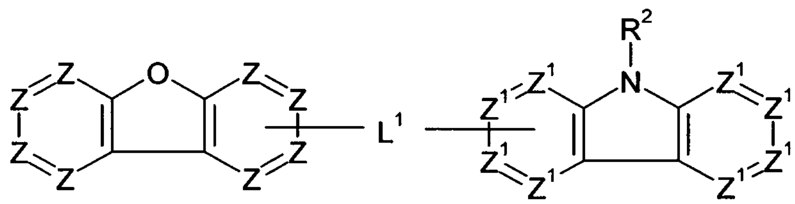

Formel (I-2-3)

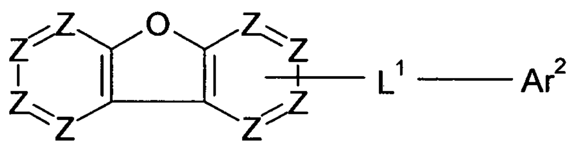

Formel (I-2-4)

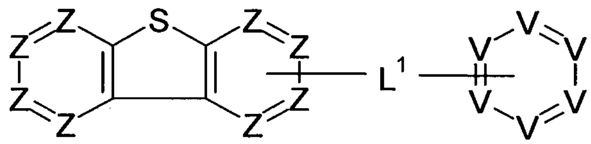

Formel (I-4-1)

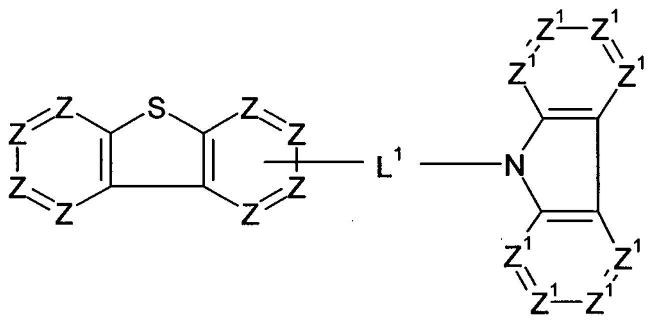

Formel (I-4-2)

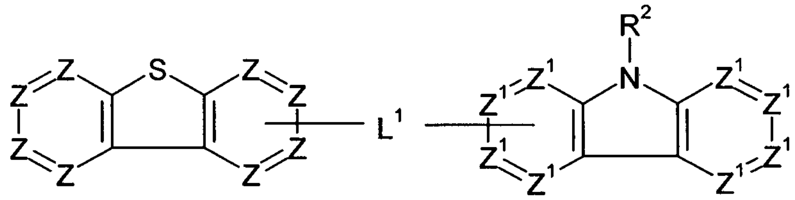

Formel (I-4-3)

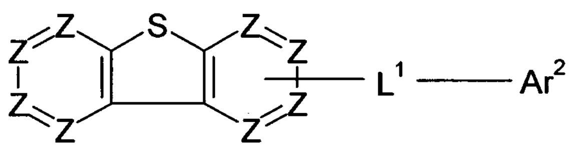

Formel (I-4-4),

wobei gilt:

V ist bei jedem Auftreten gleich oder verschieden $CR^2$, C oder N, wobei V genau dann gleich C ist, wenn eine Gruppe $L^1$ daran gebunden ist, und wobei die Maßgabe gilt, dass mindestens eine Gruppe V gleich N ist;

$Z^1$ ist bei jedem Auftreten gleich oder verschieden $CR^2$, C oder N, wobei $Z^1$ genau dann gleich C ist, wenn eine Gruppe $L^1$ daran gebunden ist;

$Ar^2$ ist eine kondensierte Arylgruppe mit 10 bis 14 aromatischen Ringatomen, die mit einem oder mehreren Resten $R^2$ substituiert sein kann;

und wobei die weiteren auftretenden Gruppen definiert sind wie oben.

Bevorzugt entsprechen die auftretenden Gruppen in Formeln (I-2-1) bis (I-2-4) und (I-4-1) bis (I-4-4) ihren oben angegebenen bevorzugten Ausführungsformen.

Weiterhin ist es bevorzugt, dass $Z^1$ gleich $CR^2$ oder C ist, wobei $Z^1$ genau dann gleich C ist, wenn eine Gruppe $L^1$ daran gebunden ist.

Weiterhin ist es bevorzugt, dass genau 1, 2 oder 3 Gruppen V im Ring gleich N sind, und die restlichen Gruppen V gleich $CR^2$ bzw. C sind. Dabei ist es bevorzugt, dass nicht mehr als zwei benachbarte Gruppen V im Ring gleich N sind, besonders bevorzugt dass keine benachbarten Gruppen V im Ring gleich N sind.

Es ist für Formeln (I-2-2), (I-2-3), (I-4-2) und (I-4-3) besonders bevorzugt, dass mindestens eine Gruppe $R^2$ als Substituent einer Gruppe $Z^1$ eine optional mit Resten $R^3$ substituierte Carbazolgruppe darstellt.

Weiterhin ist es bevorzugt, dass $Ar^2$ eine Phenanthrenylgruppe ist, die mit einem oder mehreren Resten $R^2$ substituiert sein kann.

Weiterhin ist es für Formeln (I-2-1) bis (I-2-4) und (I-4-1) bis (I-4-4) bevorzugt, dass $L^1$ eine Einfachbindung, eine Gruppe $Si(R^2)_2$ oder eine wahlweise mit Resten $R^2$ substituierte Phenylgruppe oder Biphenylgruppe ist.

Bevorzugte Ausführungsformen der Formel (II) entsprechen den folgenden Formeln (II-1) bis (II-3)

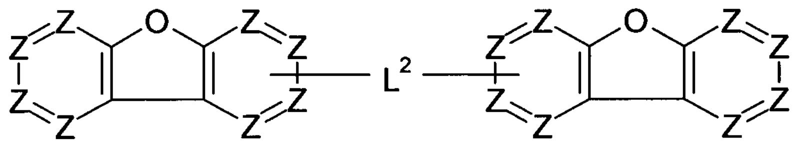

Formel (II-1)

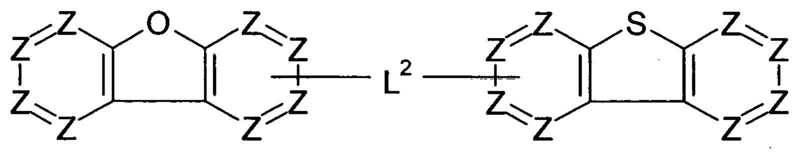

Formel (II-2)

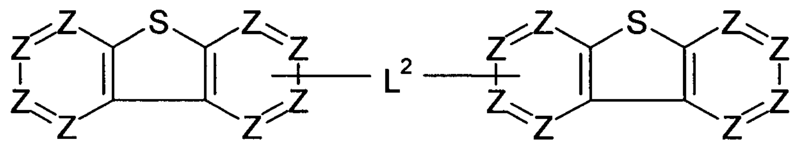

Formel (II-3),

wobei die auftretenden Gruppen definiert sind wie oben.

Bevorzugt entsprechen die auftretenden Gruppen in Formeln (II-1) bis (II-3) ihren oben angegebenen bevorzugten Ausführungsformen.

Die Gruppe $L^2$ in Formeln (II-1) bis (II-3) ist bevorzugt gewählt aus einer Einfachbindung oder Einheit der Formel (L2)

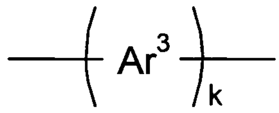

Formel (L2),

wobei gilt:

$Ar^3$ ist bei jedem Auftreten gleich oder verschieden eine Arylen- oder Heteroarylengruppe mit 6 bis 14 aromatischen Ringatomen, die mit einem oder mehreren Resten $R^2$ substituiert sein kann; und

k ist gleich 1, 2, 3 oder 4.

Bevorzugt ist dabei $Ar^3$ gewählt aus Phenyl, Pyridyl, Pyrimidyl, Naphthyl, Phenanthrenyl, Chinolinyl, Carbazolyl, Dibenzofuranyl und Dibenzothiophenyl.

Bevorzugt ist dabei k gleich 1, 2 oder 3.

Folgende Verbindungen sind Beispiele für Verbindungen M gemäß Formel (I) oder (II):

| | |
|---|---|
| 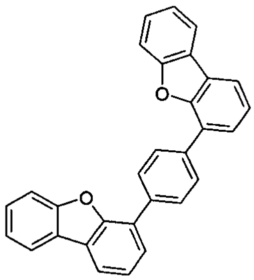 | 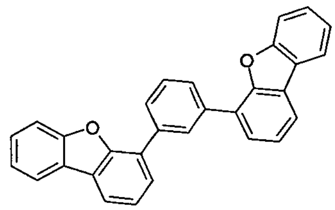 |
| (1) | (2) |
| 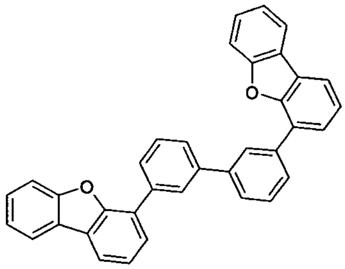 | 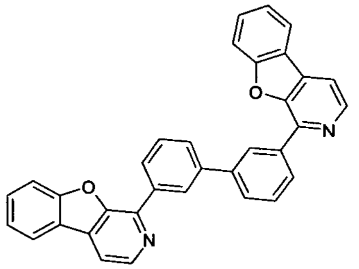 |
| (3) | (4) |
| 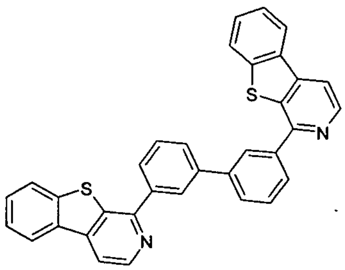 | 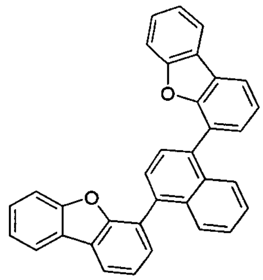 |
| (5) | (6) |
| 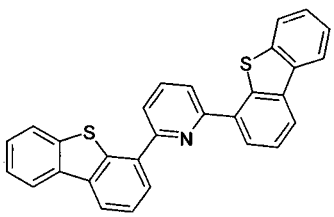 | 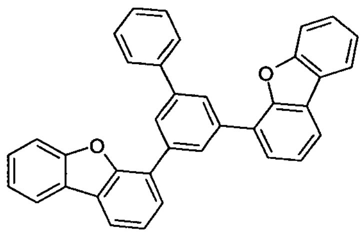 |
| (7) | (8) |
| 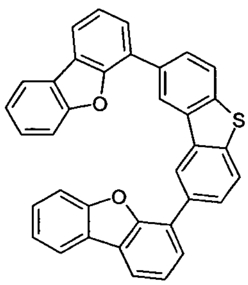 | 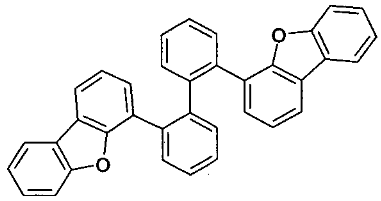 |
| (9) | (10) |

(fortgesetzt)

| | |
|---|---|
| 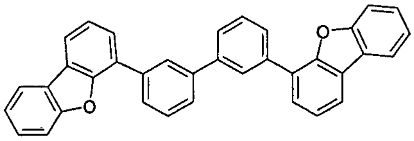 | 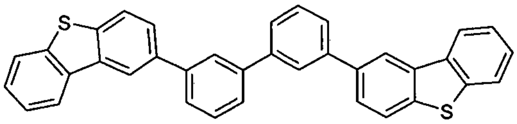 |
| (11) | (12) |
| 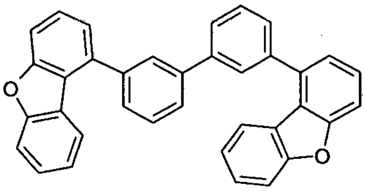 | 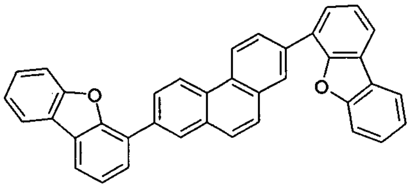 |
| (13) | (14) |
| 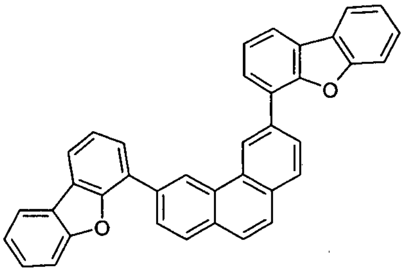 | 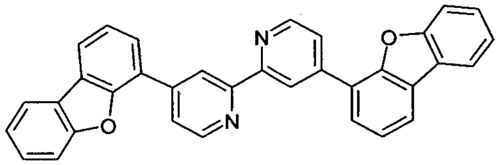 |
| (15) | (16) |
| 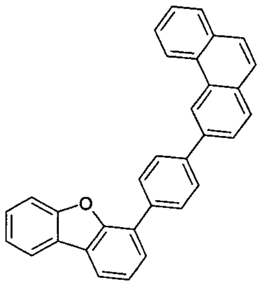 | 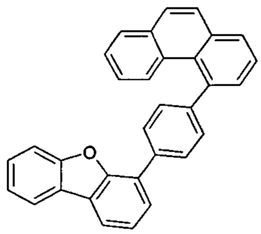 |
| (17) | (18) |
| 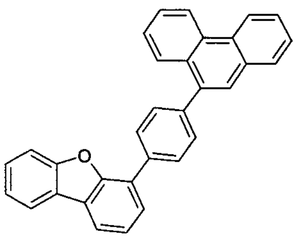 | 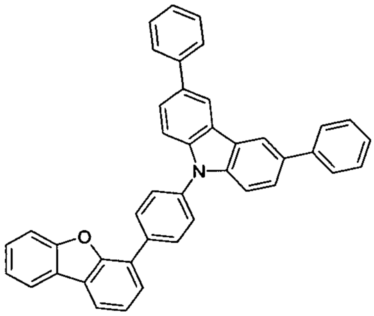 |
| (19) | (20) |

(fortgesetzt)

| | |
|---|---|
| 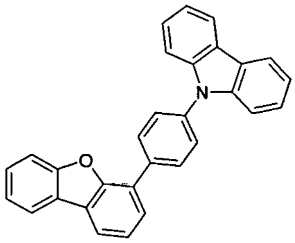 | 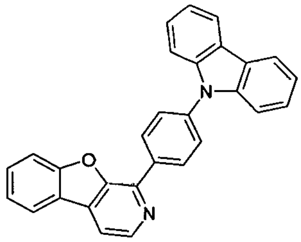 |
| (21) | (22) |
| 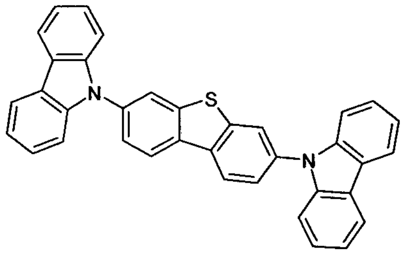 | 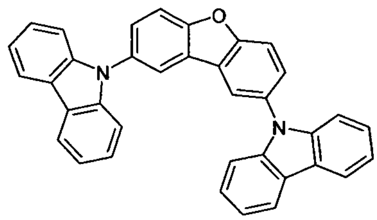 |
| (23) | (24) |
| 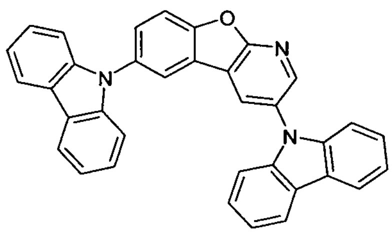 | 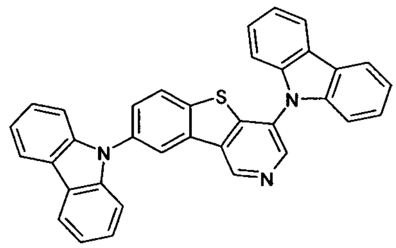 |
| (25) | (26) |
| 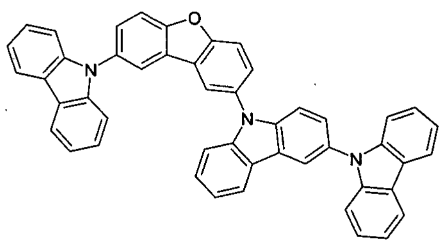 | 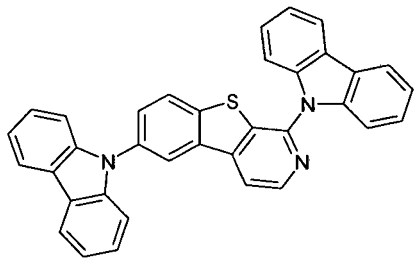 |
| (27) | (28) |
| 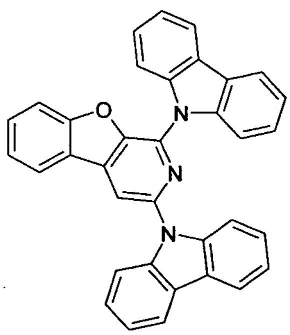 | 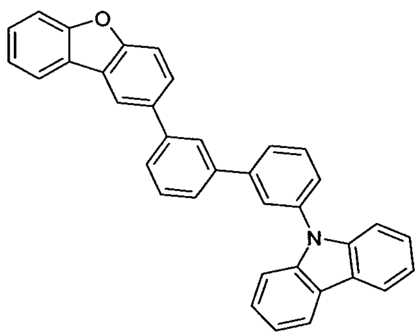 |
| (29) | (30) |

(fortgesetzt)

| | |
|---|---|
| 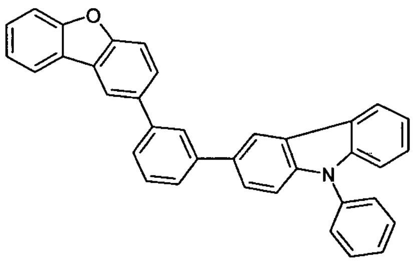 | 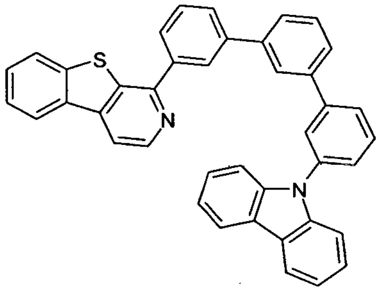 |
| (31) | (32) |
| 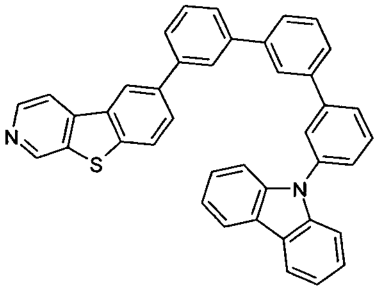 | 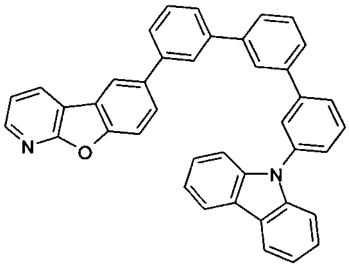 |
| (33) | (34) |
| 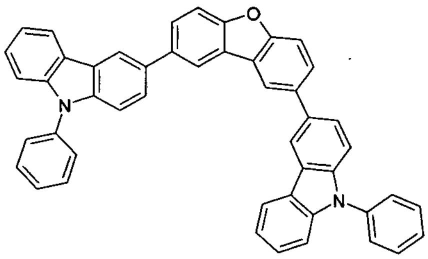 | 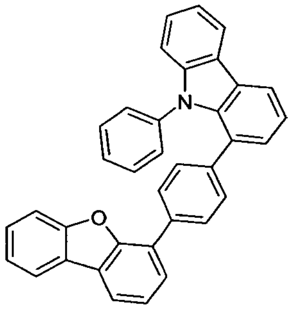 |
| (35) | (36) |
| 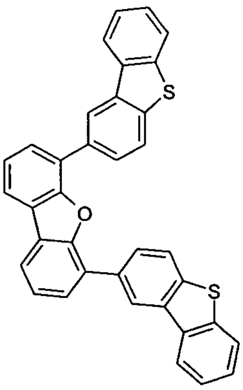 | 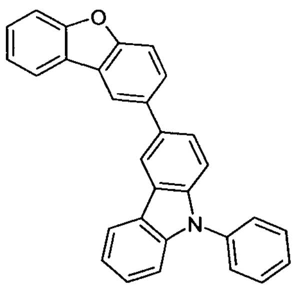 |
| (37) | (38) |

(fortgesetzt)

| | |
|---|---|
| 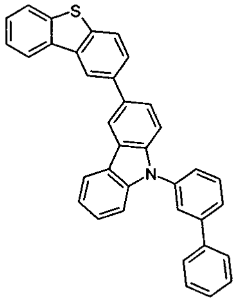 | 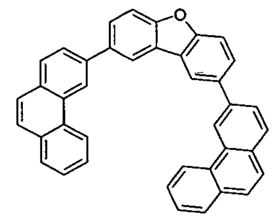 |
| (39) | (40) |
| 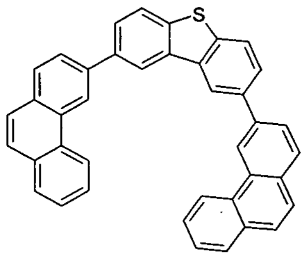 | 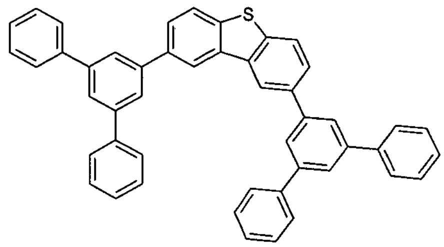 |
| (41) | (42) |
| 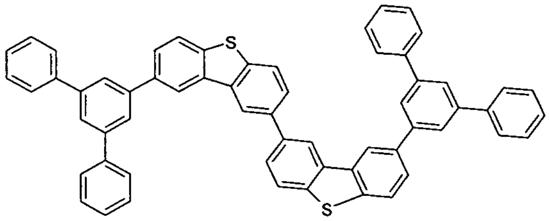 | 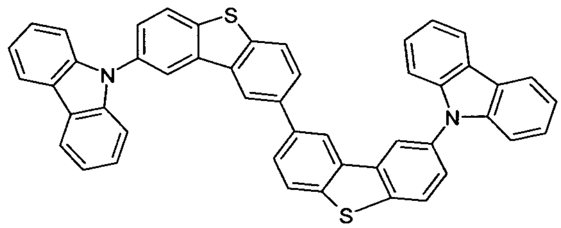 |
| (43) | (44) |
| 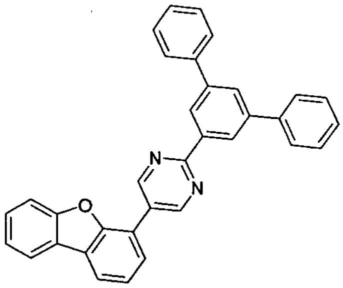 | 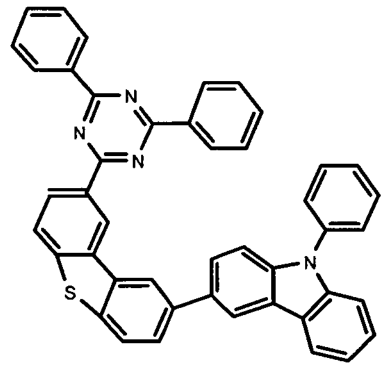 |
| (45) | (46) |
| 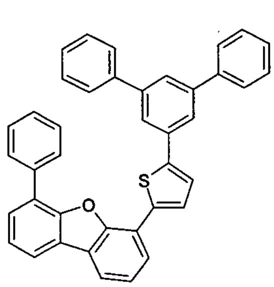 | 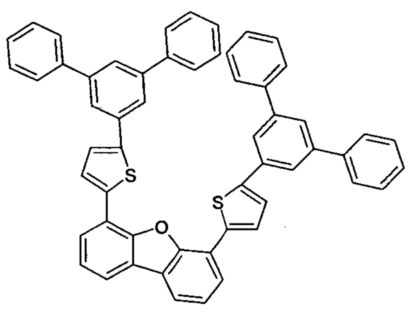 |

(fortgesetzt)

| (47) | (48) |
|---|---|
| 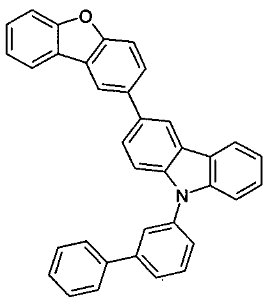 | 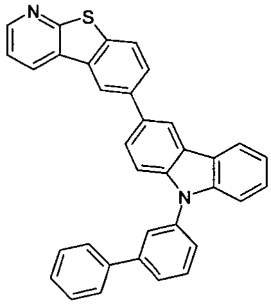 |
| (49) | (50) |
| 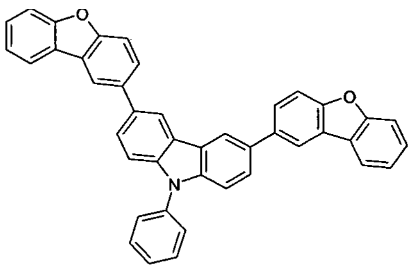 | 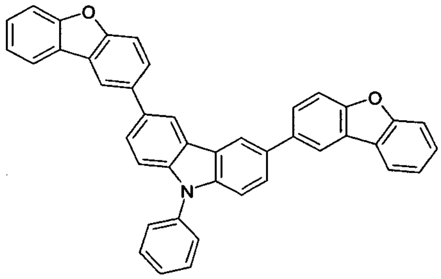 |
| (51) | (52) |
| 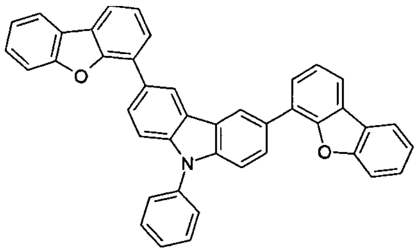 | 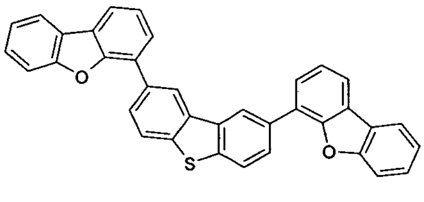 |
| (53) | (54) |
| 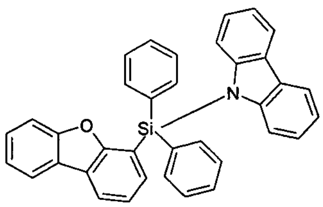 | 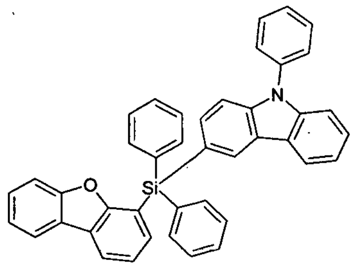 |
| (55) | (56) |
| 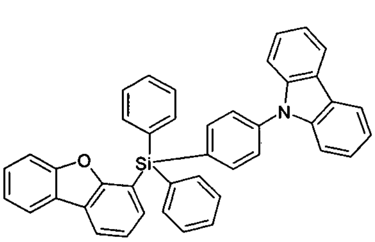 | 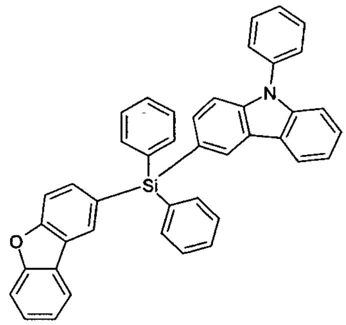 |

(fortgesetzt)

| | |
|---|---|
| (57) | (58) |
| 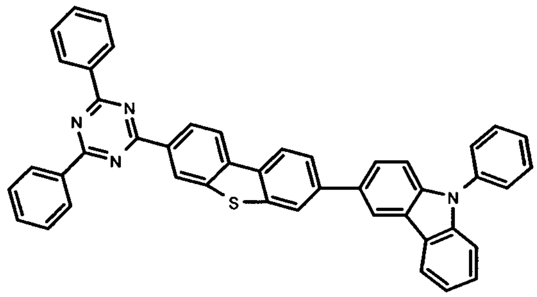 | 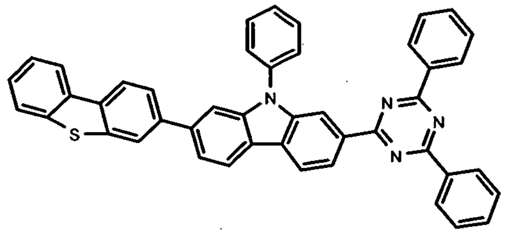 |
| (59) | (60) |
| 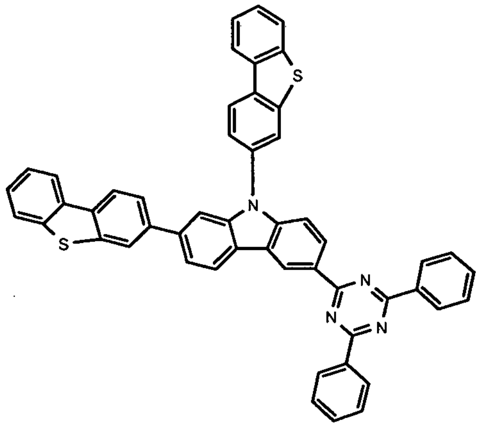 | 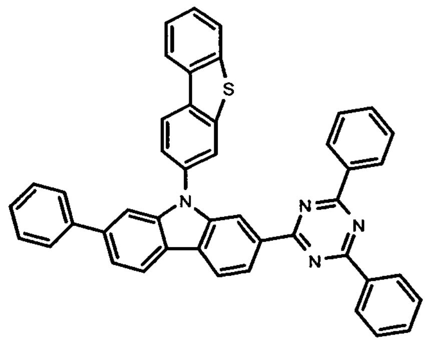 |
| (61) | (62) |
| 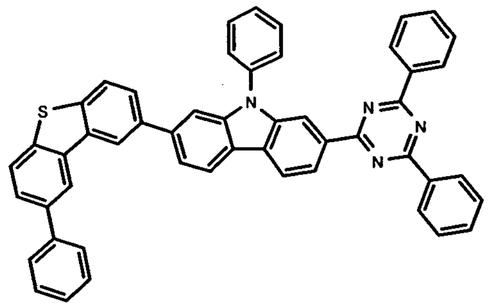 | 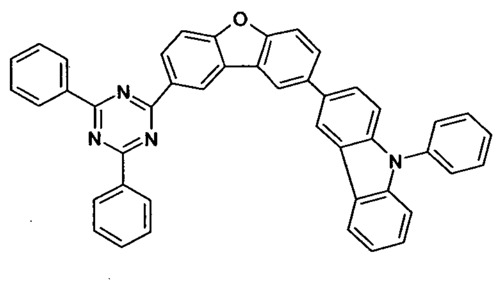 |
| (63) | (64) |
| 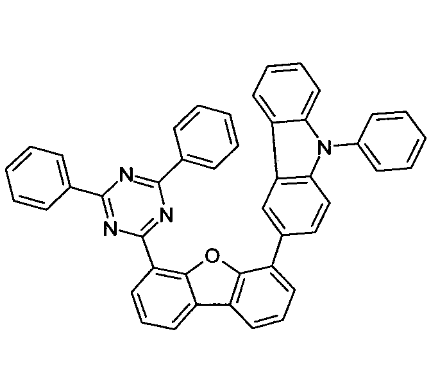 | 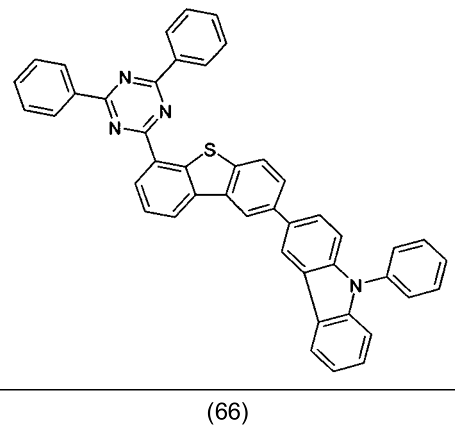 |
| (65) | (66) |

(fortgesetzt)

| | |
|---|---|
| 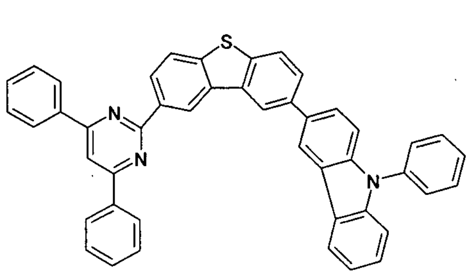 | 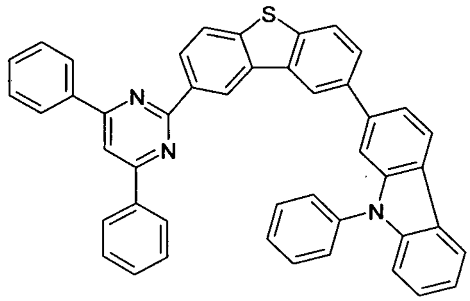 |
| (67) | (68) |
| 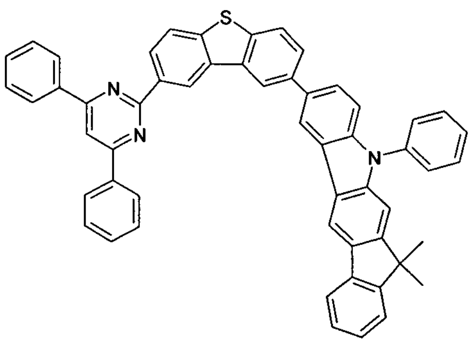 | 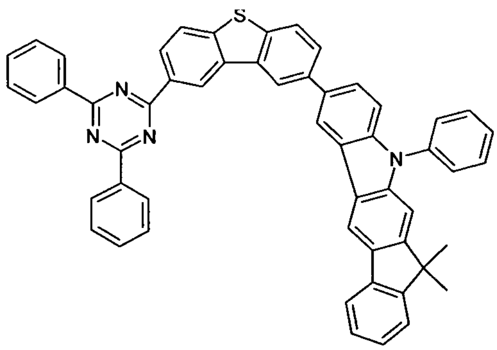 |
| (69) | (70) |
| 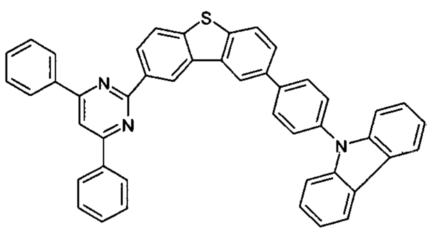 | 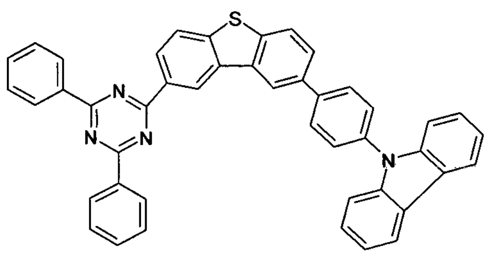 |
| (71) | (72) |
| 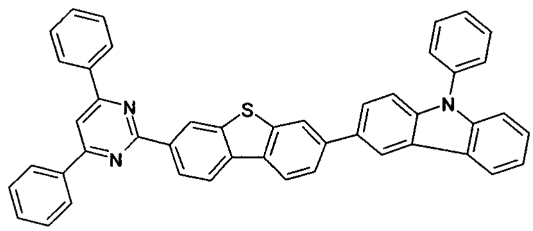 | 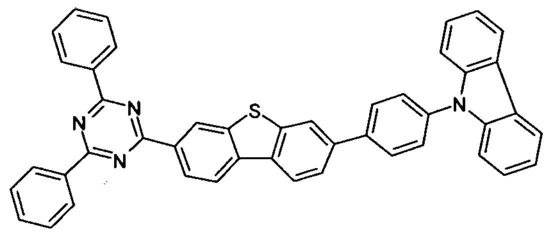 |
| (73) | (74) |
| 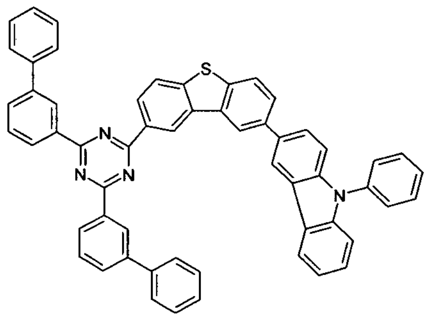 | 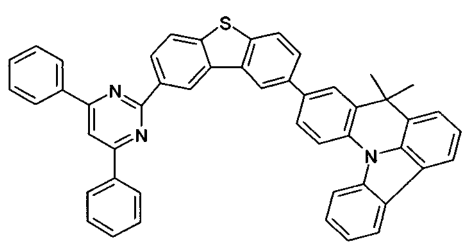 |

(fortgesetzt)

| (75) | (76) |
|---|---|
| 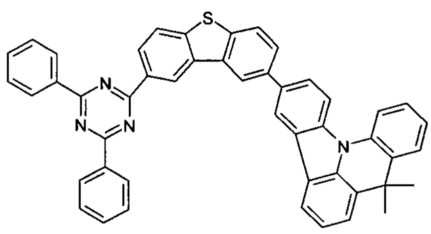 | 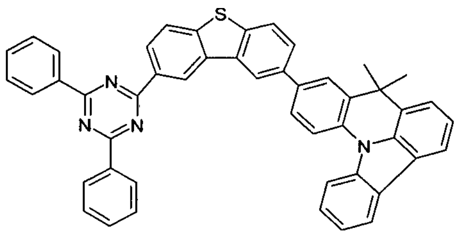 |
| (77) | (78) |
| 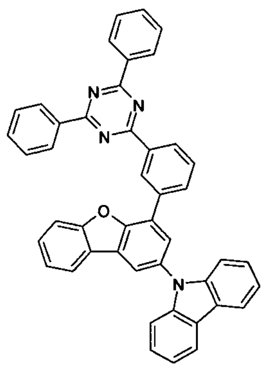 | 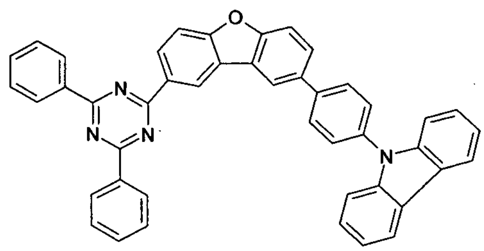 |
| (79) | (80) |
| 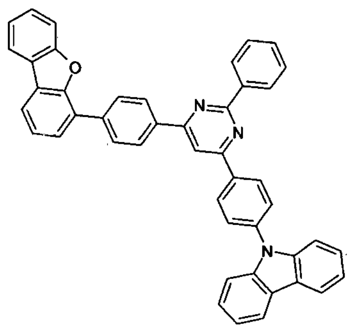 | 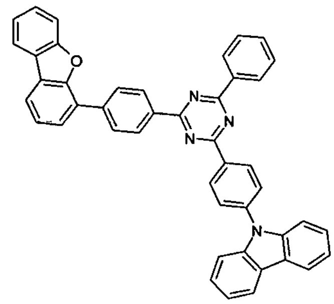 |
| (81) | (82) |
| 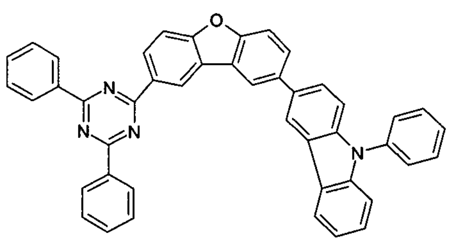 | 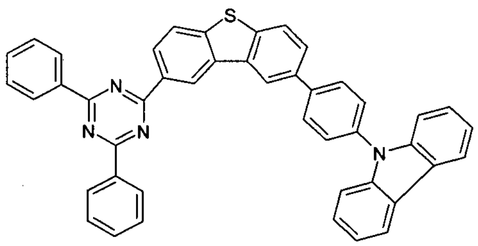 |
| (83) | (84) |
| 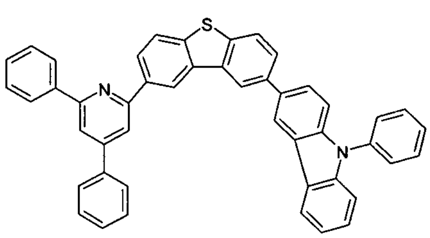 | 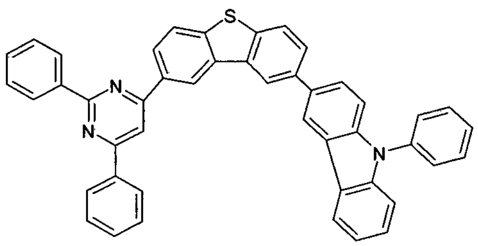 |

(fortgesetzt)

| | |
|---|---|
| (85) | (86) |
| 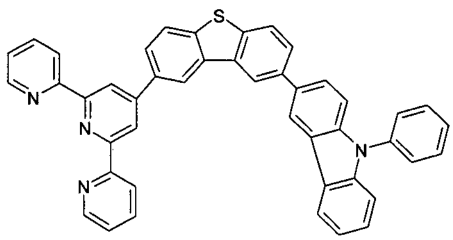 | 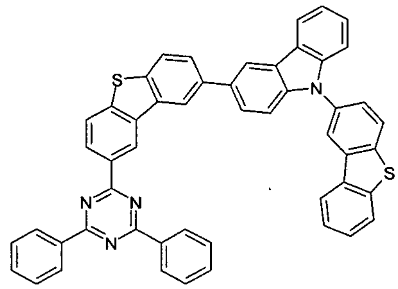 |
| (87) | (88) |
| 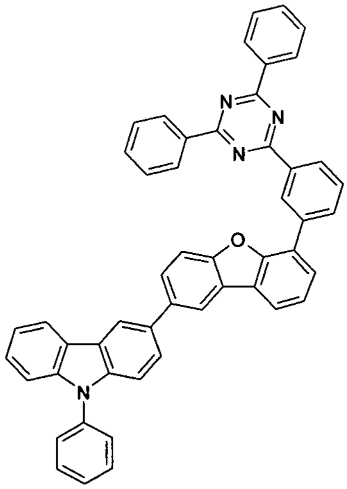 | 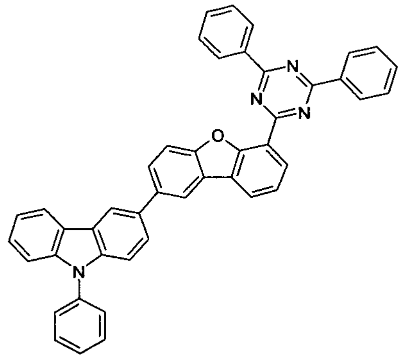 |
| (89) | (90) |
| 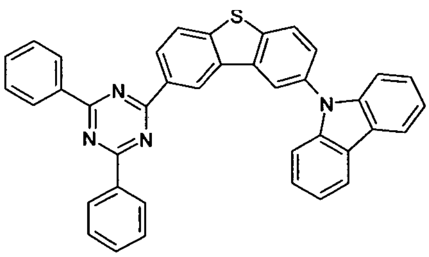 | 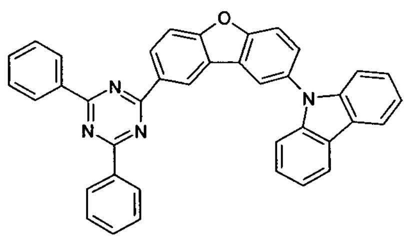 |
| (91) | (92) |
| 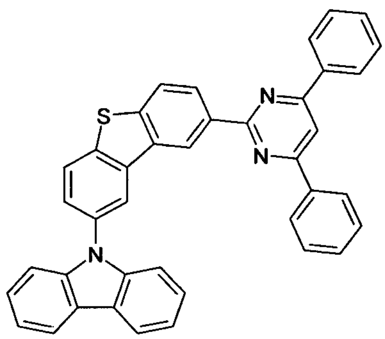 | 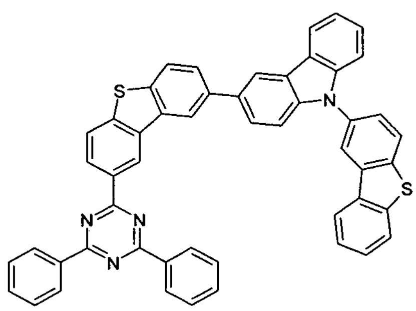 |
| (93) | (94) |

(fortgesetzt)

| | |
|---|---|
| 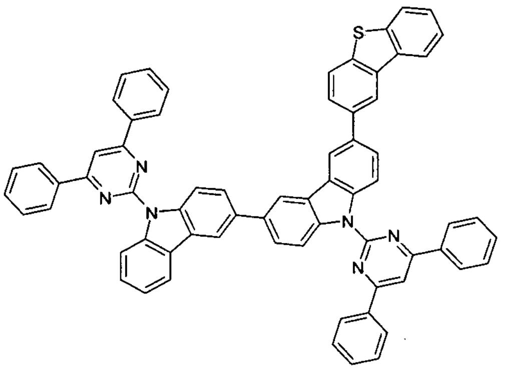 | 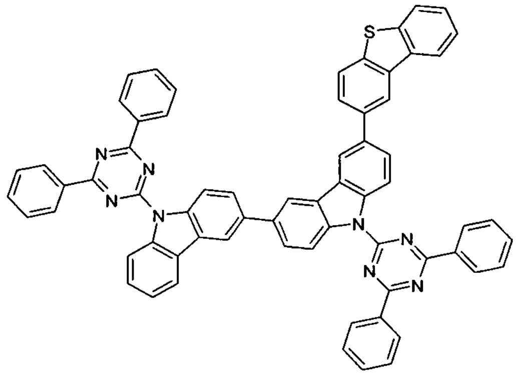 |
| (95) | (96) |
| 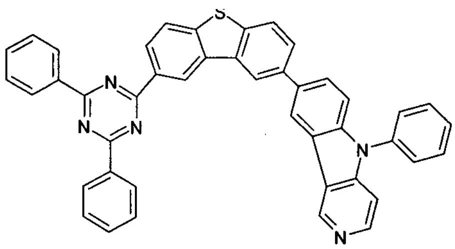 | 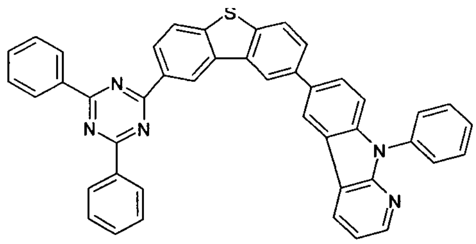 |
| (97) | (98) |
| 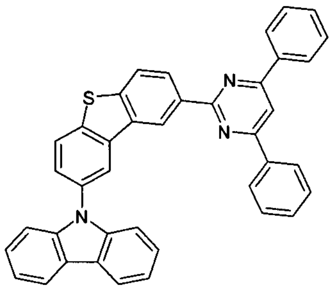 | 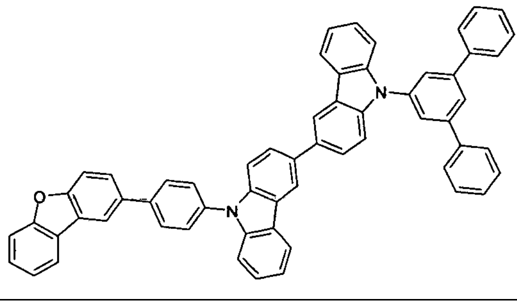 |
| (99) | (100) |
| 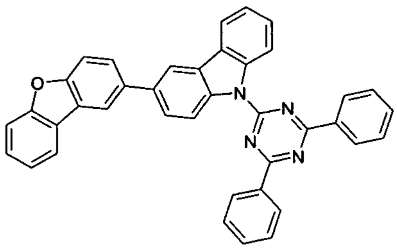 | 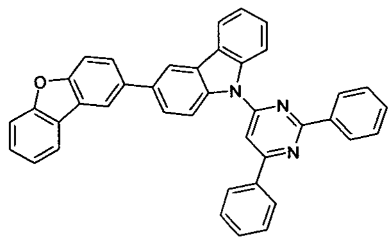 |
| (101) | (102) |
| 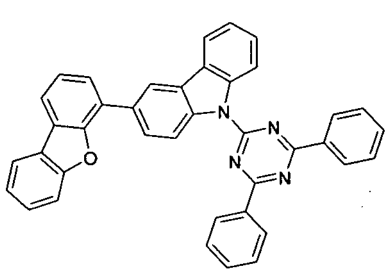 | 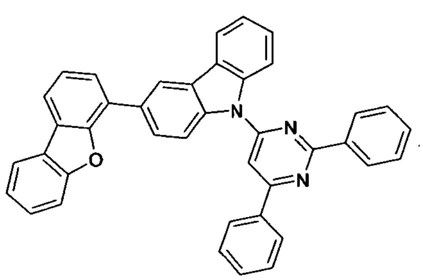 |

(fortgesetzt)

| | |
|---|---|
| (103) | (104) |
| 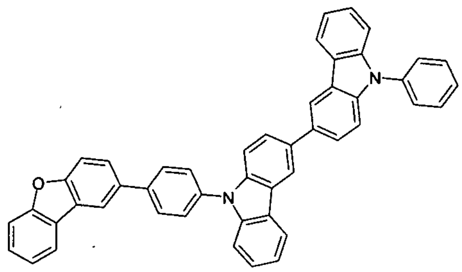 | 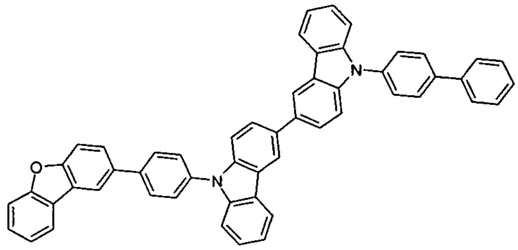 |
| (105) | (106) |
| 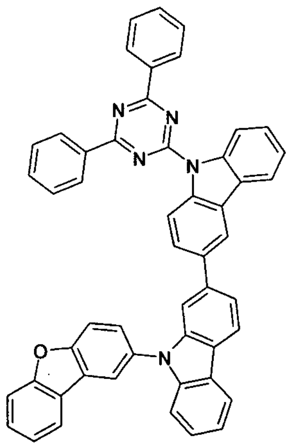 | 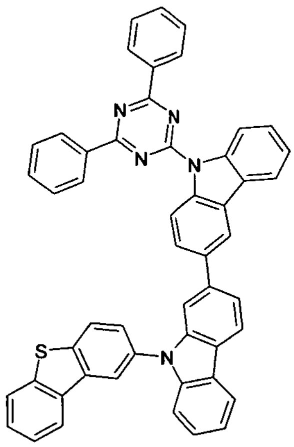 |
| (107) | (108) |
| 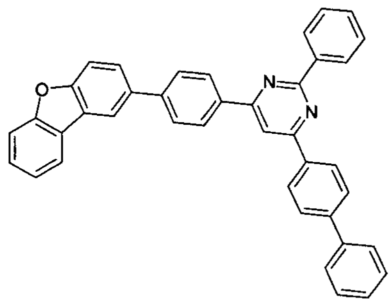 | 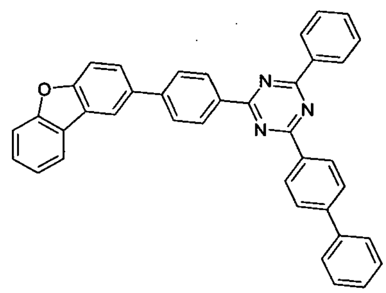 |
| (109) | (110) |
| 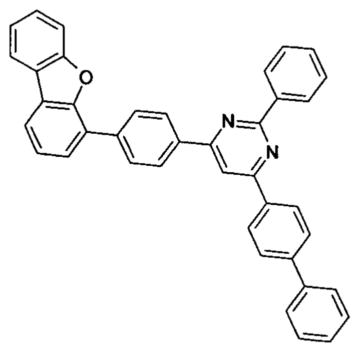 | 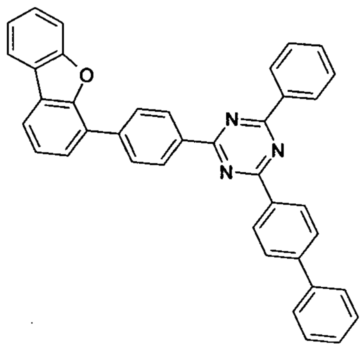 |
| (111) | (112) |

(fortgesetzt)

| | |
|---|---|
| 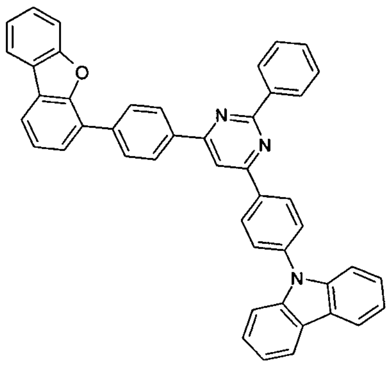 | 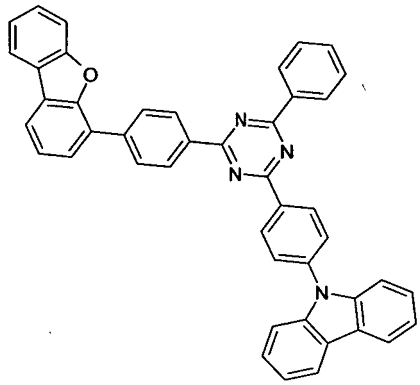 |
| (113) | (114) |

Die Verbindung M gemäß Formel (I) und (II) kann gemäß bekannten Verfahren der organischen Synthese, beispielsweise Bromierung, Buchwald-Kupplung und Suzuki-Kupplung, hergestellt werden.

Syntheseverfahren zur Herstellung der Verbindung M gemäß Formel (I) oder (II) sind beispielsweise in WO 2012/145173, WO 2012/048266, WO 2011/137072, EP 2551932, US 2009/0030202 und WO 2011/057706 detailliert beschrieben.

Im Folgenden wird die erfindungsgemäße organische Elektrolumineszenzvorrichtung näher beschrieben.

Die Verbindung M gemäß Formel (I) oder (II) ist das Matrixmaterial in der emittierenden Schicht, und die Verbindung E ist die emittierende Verbindung. Unter emittierender Verbindung wird die Verbindung verstanden, deren Emission aus der emittierenden Schicht beim Betrieb der Vorrichtung beobachtet wird. Die Verbindung M gemäß Formel (I) oder (II) trägt erfindungsgemäß nicht oder nicht wesentlich zur Emission aus der emittierenden Schicht bei.

In einer bevorzugten Ausführungsform der Erfindung besteht die emittierende Schicht im Wesentlichen aus der Verbindung M der Formel (I) oder (II) und der Verbindung E. Besonders bevorzugt besteht die emittierende Schicht ausschließlich aus der Verbindung M der Formel (I) oder (II) und der Verbindung E.

Die Verbindung E liegt bevorzugt in einem deutlich höherem Anteil in der emittierenden Schicht vor als die Verbindung M. Bevorzugt liegt der Anteil der Verbindung M zwischen 80 % und 99 %, besonders bevorzugt zwischen 90 und 98 % und ganz besonders bevorzugt zwischen 93 und 97 %. Bevorzugt liegt der Anteil der Verbindung E zwischen 1 % und 20 %, besonders bevorzugt zwischen 2 und 10 % und ganz besonders bevorzugt zwischen 3 und 7 %.

Unter Angaben von Anteilen in % wird dabei bei aus der Gasphase aufgetragenen Verbindungen der Anteil in Volumenprozent verstanden, und bei aus Lösung aufgetragenen Verbindungen der Anteil in Gewichtsprozent verstanden.

Es ist weiterhin bevorzugt, dass die Energie des $T_1$-Zustands der Verbindung M gemäß Formel (I) oder (II) ($T_1$(M)) maximal um 0.1 eV niedriger ist als die Energie des $T_1$-Zustands der Verbindung E ($T_1$(E)). Besonders bevorzugt ist $T_1(M) \geq T_1(E)$. Ganz besonders bevorzugt gilt: $T_1(M) - T_1(E) \geq 0.1$ eV, am stärksten bevorzugt $T_1(M) - T_1(E) \geq 0.2$ eV. Dabei werden die Energien der $T_1$-Zustände durch quantenchemische Rechnung bestimmt, wie in den Ausführungsbeispielen beschrieben.

Die Verbindung E ist bevorzugt eine organische Verbindung. Eine organische Verbindung im Sinne der vorliegenden Erfindung ist eine kohlenstoffhaltige Verbindung, die keine Metalle enthält. Insbesondere ist die organische Verbindung aus den Elementen C, H, D, B, Si, N, P, O, S, F, Cl, Br und I aufgebaut.

Weiterhin bevorzugt ist die Verbindung E eine lumineszente Verbindung. Eine lumineszente Verbindung im Sinne der vorliegenden Erfindung ist eine Verbindung, die in der Lage ist, unter optischer Anregung in einer Umgebung, wie sie in der organischen Elektrolumineszenzvorrichtung vorliegt, bei Raumtemperatur Licht zu emittieren. Dabei weist die Verbindung bevorzugt eine Lumineszenzquanteneffizienz von mindestens 40 % auf, besonders bevorzugt von mindestens 50 %, ganz besonders bevorzugt von mindestens 60 % und insbesondere bevorzugt von mindestens 70 %. Dabei wird die Lumineszenzquanteneffizienz bestimmt in einer Schicht in Mischung mit dem Matrixmaterial, wie sie in der organischen Elektrolumineszenzvorrichtung verwendet werden soll. Wie die Bestimmung der Lumineszenzquantenausbeute im Sinne der vorliegenden Erfindung durchgeführt wird, ist im Beispielteil beschrieben.

Weiterhin ist es bevorzugt, wenn die Verbindung E eine kurze Abklingzeit aufweist. Dabei ist die Abklingzeit bevorzugt $\leq 50$ µs. Wie die Bestimmung der Abklingzeit im Sinne der vorliegenden Erfindung durchgeführt wird, ist im Beispielteil beschrieben.

Die Energie des niedrigsten angeregten Singulettzustands ($S_1$) und des niedrigsten Triplettzustands ($T_1$) wer-

den durch quantenchemische Rechnung bestimmt. Wie diese Bestimmung im Sinne der vorliegenden Erfindung durchgeführt wird, ist im Beispielteil beschrieben.

Die betragsmäßige Differenz zwischen den Energien des $S_1$- und des $T_1$-Zustands der Verbindung E beträgt erfindungsgemäß höchstens 0.15 eV. Bevorzugt ist die betragsmäßige Differenz $\leq$ 0.10 eV, besonders bevorzugt $\leq$ 0.08 eV, ganz besonders bevorzugt $\leq$ 0.05 eV.

Die Verbindung E ist eine aromatische Verbindung, die sowohl mindestens einen Donor- als auch mindestens einen Akzeptorsubstituenten aufweist, wobei das LUMO und das HOMO der Verbindung räumlich nur geringfügig überlappen. Was unter Donor- bzw. Akzeptorsubstituenten verstanden wird, ist dem Fachmann prinzipiell bekannt. Die Donorsubstituenten sind Diaryl- bzw. Diheteroarylaminogruppen sowie Carbazolgruppen bzw. Carbazolderivate, die jeweils bevorzugt über N an die aromatische Verbindung gebunden sind. Dabei können diese Gruppen auch weiter substituiert sein. Die Akzeptorsubstituenten sind Cyanogruppen und elektronenarme Heteroarylgruppen, die auch weiter substituiert sein können.

Es ist bevorzugt, dass für das LUMO der Verbindung E (LUMO(E)), und das HOMO der Verbindung M (HOMO(M)) gilt:

$$\mathrm{LUMO(E)} - \mathrm{HOMO(M)} > S_1(\mathrm{E}) - 0.4\ \mathrm{eV};$$

besonders bevorzugt:

$$\mathrm{LUMO(E)} - \mathrm{HOMO(M)} > S_1(\mathrm{E}) - 0.3\ \mathrm{eV};$$

und ganz besonders bevorzugt:

$$\mathrm{LUMO(E)} - \mathrm{HOMO(M)} > S_1(\mathrm{E}) - 0.2\ \mathrm{eV}.$$

Dabei ist $S_1$(E) der erste angeregte Singulettzustand $S_1$ der Verbindung E. Dabei werden die genannten HOMO- und LUMO-Energien LUMO(E) und HOMO(M) durch quantenchemische Rechnungen bestimmt, wie in den Ausführungsbeispielen beschrieben.

Beispiele für Verbindungen E zur Verwendung in der erfindungsgemäßen Vorrichtung sind in der folgenden Tabelle aufgeführt.

| | | |
|---|---|---|
| 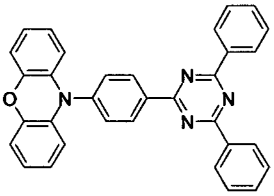 | 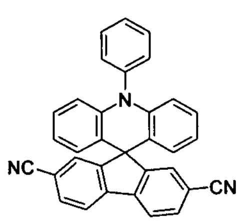 | 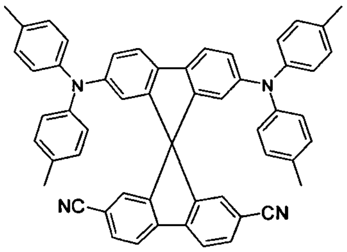 |
| 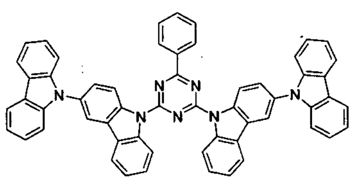 | 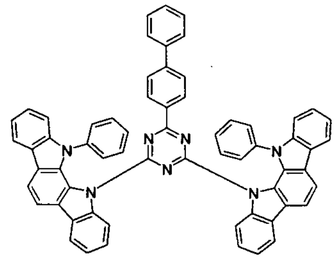 | 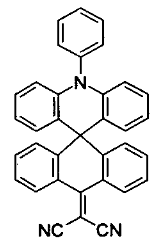 |
| 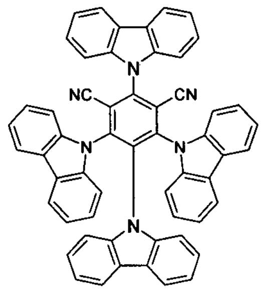 | 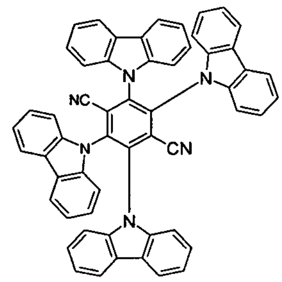 | 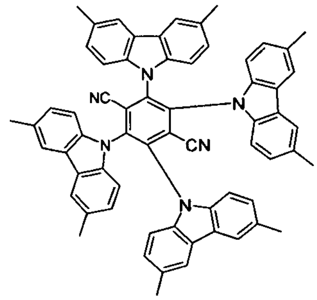 |
| 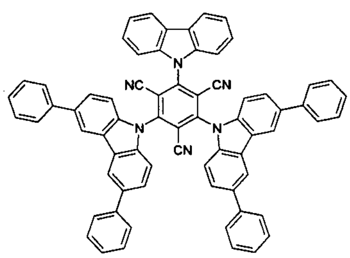 | 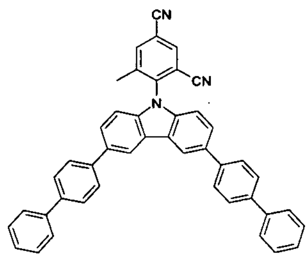 | 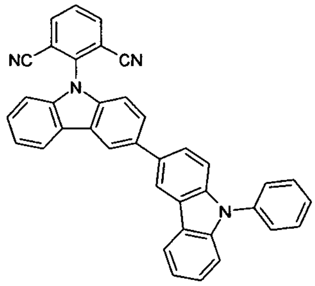 |
| 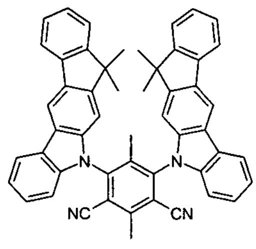 | 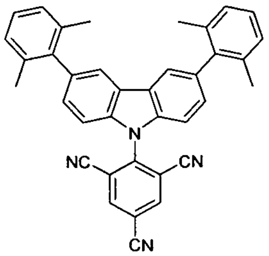 | 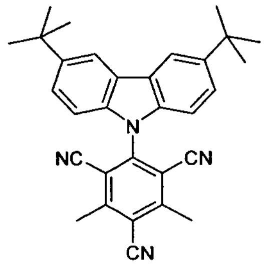 |

| | | |
|---|---|---|
| 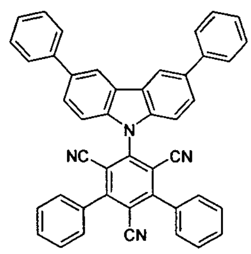 | 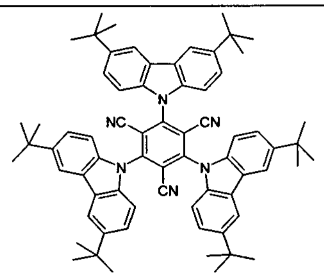 | 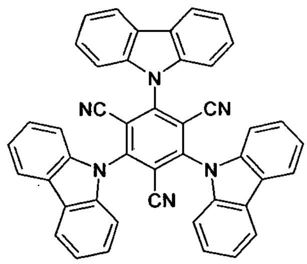 |

Die organische Elektrolumineszenzvorrichtung enthält Kathode, Anode und emittierende Schicht. Außer diesen Schichten kann sie noch weitere Schichten enthalten, beispielsweise Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Bevorzugt enthält sie eine oder mehrere Lochtransportschichten, welche zwischen Anode und emittierender Schicht angeordnet sind, und eine oder mehrere Elektronentransportschichten, welche zwischen Kathode und emittierender Schicht angeordnet sind.

In den weiteren Schichten der erfindungsgemäßen organischen Elektrolumineszenzvorrichtung, insbesondere in den Lochinjektions- und -transportschichten und in den Elektroneninjektions- und -transportschichten, können alle Materialien verwendet werden, die für diese Verwendung im Stand der Technik bekannt sind.

Dabei können die Lochtransportschichten auch p-dotiert bzw. die Elektronentransportschichten auch n-dotiert sein. Unter einer p-dotierten Schicht wird eine Schicht verstanden, in der durch einen p-Dotanden freie Löcher erzeugt werden und deren Leitfähigkeit dadurch erhöht ist. Eine umfassende Diskussion von dotierten Transportschichten in OLEDs findet sich in Chem. Rev. 2007, 107, 1233. Besonders bevorzugt ist der p-Dotand in der Lage, das Lochtransportmaterial in der Lochtransportschicht zu oxidieren, hat also ein ausreichend hohes Redoxpotential, insbesondere ein höheres Redoxpotential als das Lochtransportmaterial. Als p-Dotanden sind prinzipiell alle Verbindungen geeignet, welche Elektronenakzeptorverbindungen darstellen und die Leitfähigkeit der organischen Schicht durch Oxidation des Lochtransportmaterials erhöhen können. Der Fachmann kann im Rahmen seines allgemeinen Fachwissens ohne größeren Aufwand geeignete Verbindungen identifizieren. Insbesondere geeignet als Dotanden sind die in WO 2011/073149, EP 1968131, EP 2276085, EP 2213662, EP 1722602, EP 2045848, DE 102007031220, US 8044390, US 8057712, WO 2009/003455, WO 2010/094378, WO 2011/120709 und US 2010/0096600 offenbarten Verbindungen.

Als Kathode der organischen Elektrolumineszenzvorrichtung sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lanthanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag oder Al, wobei dann in der Regel Kombinationen der Metalle, wie beispielsweise Ca/Ag, Mg/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, $Li_2O$, $BaF_2$, MgO, NaF, CsF, $Cs_2CO_3$, etc.). Weiterhin kann dafür Lithiumchinolinat (LiQ) verwendet werden. Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektroden (z. B. Al/Ni/$NiO_x$, Al/$PtO_x$) bevorzugt sein. Für einige Anwendungen muss mindestens eine der Elektroden transparent oder teiltransparent sein, um entweder die Bestrahlung des organischen Materials (organische Solarzelle) oder die Auskopplung von Licht (OLED, O-LASER) zu ermöglichen. Bevorzugte Anodenmaterialien sind hier leitfähige gemischte Metalloxide. Besonders bevorzugt sind Indium-ZinnOxid (ITO) oder Indium-Zink Oxid (IZO). Bevorzugt sind weiterhin leitfähige, dotierte organische Materialien, insbesondere leitfähige dotierte Polymere. Weiterhin kann die Anode auch aus mehreren Schichten bestehen, beispielsweise aus einer inneren Schicht aus ITO und einer äußeren Schicht aus einem Metalloxid, bevorzugt Wolframoxid, Molybdänoxid oder Vanadiumoxid.

Die Vorrichtung wird entsprechend (je nach Anwendung) strukturiert, kontaktiert und schließlich versiegelt, um schädigende Effekte von Wasser und Luft auszuschließen.

In einer bevorzugten Ausführungsform ist die organische Elektrolumineszenzvorrichtung dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner $10^{-5}$ mbar, bevorzugt kleiner $10^{-6}$ mbar aufgedampft. Dabei ist es jedoch auch möglich, dass der Anfangsdruck noch geringer ist, beispielsweise kleiner $10^{-7}$ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen $10^{-5}$ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organischen Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck, Nozzle Printing oder Offsetdruck, besonders bevorzugt aber LITI (Light Induced Thermal Imaging, Thermotransferdruck) oder Ink-Jet Druck (Tintenstrahldruck), hergestellt werden. Hierfür sind lösliche Verbindungen gemäß Formel (I) oder (II) nötig. Hohe Löslichkeit lässt sich durch geeignete Substitution der Verbindungen erreichen.

Weiterhin bevorzugt ist es, dass zur Herstellung der organischen Elektrolumineszenzvorrichtung eine oder mehrere Schichten aus Lösung und eine oder mehrere Schichten durch ein Sublimationsverfahren aufgetragen werden.

Ein weiterer Gegenstand der vorliegenden Erfindung ist daher ein Verfahren zur Herstellung einer erfindungsgemäßen organischen Elektrolumineszenzvorrichtung, dadurch gekennzeichnet, dass mindestens eine Schicht mit einem Sublimationsverfahren aufgebracht wird und/oder dass mindestens eine Schicht mit einem OVPD (Organic Vapour Phase Deposition) Verfahren oder mit Hilfe einer Trägergassublimation aufgebracht wird und/oder dass mindestens eine Schicht aus Lösung, durch Spincoating oder mit einem Druckverfahren aufgebracht wird.

Die folgenden Ausführungsbeispiele dienen der näheren Erläuterung der Erfindung und ihrer technischen Effekte und sind nicht beschränkend auszulegen.

## Ausführungsbeispiele

### A) Bestimmung von HOMO, LUMO, Singulett- und Triplettniveau

Die HOMO- und LUMO-Energieniveaus sowie die Energie des niedrigsten Triplettzustands $T_1$ bzw. des niedrigsten angeregten Singulettzustands $S_1$ der Materialien werden über quantenchemische Rechnungen bestimmt. Hierzu wird das Programmpaket "Gaussian09W" (Gaussian Inc.) verwendet. Zur Berechnung organischer Substanzen ohne Metalle (in Tabelle 1 mit Methode "org." bezeichnet) wird zuerst eine Geometrieoptimierung mit der Methode "Ground State/Semi-empirical/Default Spin/AM1/Charge 0/Spin Singlet" durchgeführt. Im Anschluss erfolgt auf Grundlage der optimierten Geometrie eine Energierechnung. Hierbei wird die Methode "TD-SFC/DFT/Default Spin/B3PW91" mit dem Basissatz "6-31G(d)" verwendet (Charge 0, Spin Singlet). Für metallhaltige Verbindungen (in Tabelle 1 mit Methode "M-org." bezeichnet) wird die Geometrie über die Methode "Ground State/ Hartree-Fock/Default Spin/LanL2MB/Charge 0/Spin Singlet" optimiert. Die Energierechnung erfolgt analog zu den organischen Substanzen, wie oben beschrieben, mit dem Unterschied, dass für das Metallatom der Basissatz "LanL2DZ" und für die Liganden der Basissatz "6-31G(d)" verwendet wird. Aus der Energierechnung erhält man das HOMO-Energieniveau HEh bzw. LUMO-Energieniveau LEh in Hartree-Einheiten. Daraus werden die anhand von Cyclovoltammetriemessungen kalibrierten HOMO- und LUMO-Energieniveaus in Elektronenvolt wie folgt bestimmt:

$$\text{HOMO(eV)} = ((\text{HEh}*27.212)-0.9899)/1.1206$$

$$\text{LUMO(eV)} = ((\text{LEh}*27.212)-2.0041)/1.385$$

Diese Werte sind im Sinne dieser Anmeldung als HOMO- bzw. LUMO-Energieniveaus der Materialien anzusehen.

Der niedrigste Triplettzustand $T_1$ ist definiert als die Energie des Triplettzustands mit der niedrigsten Energie, der sich aus der beschriebenen quantenchemischen Rechnung ergibt.

Der niedrigste angeregte Singulettzustand $S_1$ ist definiert als die Energie des angeregten Singulettzustands mit der niedrigsten Energie, der sich aus der beschriebenen quantenchemischen Rechnung ergibt.

In Tabelle 1 sind die HOMO- und LUMO-Energieniveaus sowie $S_1$ und $T_1$ der verschiedenen Materialien angegeben.

Tabelle 1: HOMO, LUMO, $T_1$, $S_1$ der relevanten Materialien

| Material | Methode | HOMO (eV) | LUMO (eV) | $S_1$ (eV) | $T_1$ (eV) |
|---|---|---|---|---|---|
| D1 | org. | -6.11 | -3.40 | 2.50 | 2.41 |

### B) Bestimmung der PL-Quanteneffizienz (PLQE)

Von den in den verschiedenen OLEDs verwendeten Emissionsschichten wird ein 50 nm dicker Film auf ein geeignetes transparentes Substrat, vorzugsweise Quarz, aufgebracht, d. h. die Schicht enthält dieselben Materialien in derselben Konzentration wie in der OLED. Hierbei werden die gleichen Herstellungsbedingungen wie bei der Herstellung der Emissionsschicht für die OLEDs verwendet. Von diesem Film wird ein Absorptionsspektrum im Wellenlängenbereich von 350-500 nm gemessen. Hierzu wird das Reflexionsspektrum $R(\lambda)$ sowie das Transmissionsspektrum $T(\lambda)$ der Probe unter einem Einfallswinkel von 6° (also nahezu senkrechter Einfall) bestimmt. Als Absorptionsspektrum im Sinne dieser

Anmeldung wird A(λ)=1-R(λ)-T(λ) definiert.

Gilt A(λ) ≤ 0.3 im Bereich 350-500nm, so wird die zum Maximum des Absorptionsspektrums gehörige Wellenlänge im Bereich 350-500 nm als $\lambda_{exc}$ definiert. Gilt für irgendeine Wellenlänge A(λ) > 0.3, so wird als $\lambda_{exc}$ die größte Wellenlänge definiert, bei der A(λ) von einem Wert kleiner 0.3 zu einem Wert größer 0.3 oder von einem Wert größer 0.3 zu einem Wert kleiner 0.3 wechselt.

Zur Bestimmung der PLQE wird ein Messplatz Hamamatsu C9920-02 verwendet. Das Prinzip beruht auf der Anregung der Probe mit Licht definierter Wellenlänge und der Messung der absorbierten und emittierten Strahlung. Die Probe befindet sich während der Messung in einer Ulbrichtkugel ("integrating sphere"). Das Spektrum des Anregungslichts ist in etwa gaußförmig mit einer Halbwertsbreite < 10 nm und Peakwellenlänge $\lambda_{exc}$ wie oben definiert.

Die PLQE wird nach dem für den genannten Messplatz üblichen Auswerteverfahren bestimmt. Es ist strengstens darauf zu achten, dass die Probe zu keinem Zeitpunkt mit Sauerstoff in Berührung kommt, da die PLQE von Materialien mit kleinem energetischen Abstand zwischen $S_1$ und $T_1$ durch Sauerstoff sehr stark reduziert wird (H. Uoyama et al., Nature 2012, Vol. 492, 234).

In Tabelle 2 ist die PLQE für die Emissionsschichten der OLEDs wie oben definiert zusammen mit der verwendeten Anregungswellenlänge angegeben.

**C) Bestimmung der Abklingzeit**

Zur Bestimmung der Abklingzeit wird eine Probe verwendet, die wie oben unter "Bestimmung der PL-Quanteneffizienz (PLQE)" beschrieben hergestellt wurde. Die Messung erfolgt im Vakuum. Die Probe wird bei Raumtemperatur durch einen Laserpuls geeigneter Intensität angeregt (Wellenlänge 266 nm, Pulsdauer ca. 1.5 ns). Nach der Anregung (definiert als t = 0) wird der zeitliche Verlauf der emittierten Photolumineszenz gemessen. Für die Messdaten ab dem Zeitpunkt t = 250 ns wird die Abklingzeit $t_a = t_e$ -250 ns bestimmt. Dabei ist $t_e$ derjenige Zeitpunkt nach t = 250 ns, bei dem die Intensität erstmals auf 1/e ihres Wertes bei t = 250 ns abgefallen ist.

**D) Synthese von Verbindungen**

Die meisten der den folgenden Beispielen verwendeten Verbindungen sind im Stand der Technik bekannt. Beispielsweise ist die Synthese des Dotanden D1 in Uoyama et al., Nature 2012, Vol. 492, 234 offenbart. Die Synthese der Hostmaterialien H1, H2 und H3 ist in WO 2011/057706, und die Synthese von H4 ist in WO 2012/048266 offenbart. Die Synthese von H5 ist in WO 2012/12145173 offenbart, die Synthese von H6 ist in US 2009/0030202 offenbart.

Die Synthese von H7 ist im Folgenden beschrieben:

**Schritt 1: Synthese von 2-Bromo-8-[1,1';3',1"]terphenyl-5'-yl-dibenzothiophen**

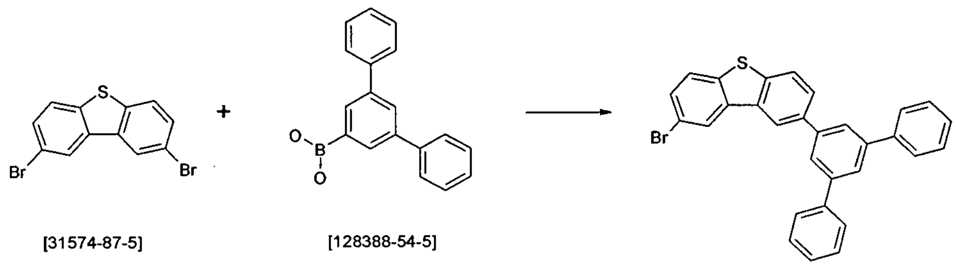

[31574-87-5] [128388-54-5]

Eine gut gerührte Suspension von 15 g (40 mmol) 2,8-Dibromodibenzothiophen, 11 g (40 mmol) (3,5-Diphenylphenyl)-boronsäure und 63,9 g (127 mmol) $Na_2CO_3$ in 500 ml DMF wird mit 2,47 g (8,1 mmol) Tetrakis-triphenylphosphinopalladium(0) versetzt und anschließend 16 h unter Rückfluss erhitzt. Nach Erkalten wird der ausgefallene Feststoff abgesaugt, dreimal mit 50 ml Toluol, dreimal mit 50 ml Ethanol: Wasser (1:1, v:v) und dreimal mit 100 ml Ethanol gewaschen. Dann wird dreimal aus DMF (ca. 15 ml / g) umkristallisiert. Dabei werden 12,5 g (25 mmol) Produkt (88.0 % d. Th.) in einer Reinheit von 99.9 % (HPLC) erhalten.

**Schritt 2: Synthese von 2-(8-[1,1';3',1"]Terphenyl-5'-yl-dibenzothiophen-2-yl)-boronsäure**

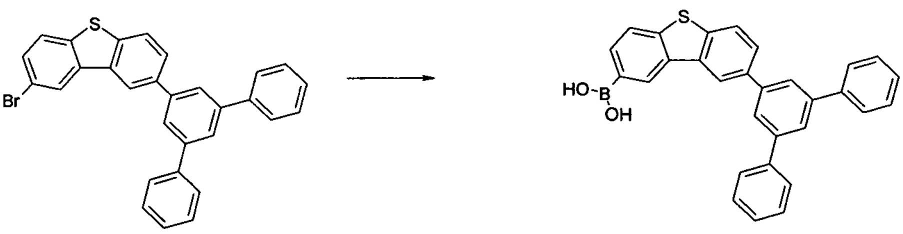

Eine Suspension von 49 g (100 mmol)) 2-Bromo-8-[1,1';3',1"]terphenyl-5'-yl-dibenzothiophen in 1000 ml THF wird bei -78 °C unter gutem Rühren tropfenweise mit 52 ml (130 mmol) n-Buthyllithium (2.5 M in n-Hexan) versetzt und 2 h nachgerührt. Die rote Lösung wird unter gutem Rühren auf ein Mal mit 16.7 ml (150 mmol) Trimethylborat versetzt, 30 min. bei -78 °C nachgerührt, dann während 3 h auf Raumtemperatur erwärmt, mit 300 ml Wasser versetzt und 30 min. gerührt. Die organische Phase wird abgetrennt und im Vakuum zur Trockene eingeengt. Der Feststoff wird in 100 ml n-Hexan aufgenommen, abgesaugt, einmal mit 100 ml Hexan gewaschen und im Vakuum getrocknet. Ausbeute: 37 g (81 mmol), 83 %, Reinheit ca. 90 %ig (NMR) an Boronsäure, mit wechselnden Mengen an Boronsäureanhydrid und Boronsäure. Die Boronsäure kann ohne weitere Reinigung im nächsten Schritt eingesetzt werden.

**Schritt 3: Synthese von 8,8'-Bis-[1,1';3',1"]terphenyl-5'-yl-[2,2']bi[dibenzothiophenyl]**

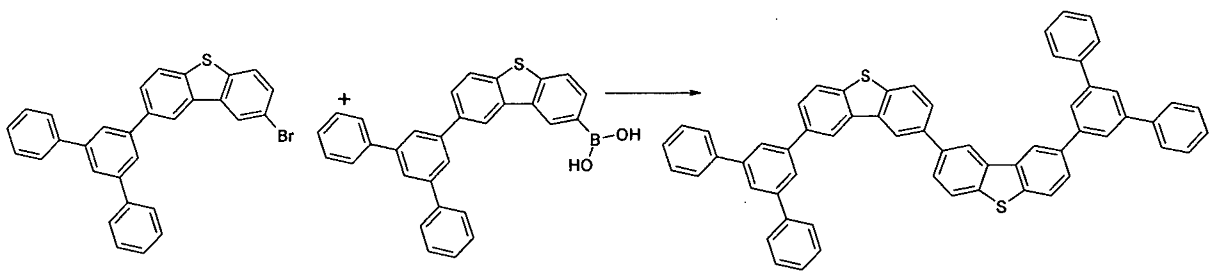

66.7 g (136 mmol) 2-Bromo-8-[1,1';3',1"]terphenyt-5'-yl-dibenzothiophen, 65,6 g (144 mmol) 2-(8-[1,1';3',1"] Terphenyl-5'-yl-dibenzothiophen-2-yl)-boronsäure und 78,9 ml (158 mmol) $Na_2CO_3$ (2M-Lösung) werden in 120 mL Toulol, 120 mL Ethanol und 100 mL Wasser suspendiert. Zu dieser Suspension werden 2,6 g (2.2 mmol) $Pd(PPh_3)_4$ gegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Der Rückstand wird aus Toluol umkristallisiert und abschließend im Hochvakuum (p = 5 x $10^{-5}$ mbar) sublimiert. Die Ausbeute beträgt 105 g (130 mmol), entsprechend 96 % der Theorie.

**E) Herstellung von OLEDs**

In den folgenden Beispielen V1 und E1 bis E7 (siehe Tabellen 1 und 2) werden die Daten verschiedener OLEDs vorgestellt.

Glasplättchen, die mit strukturiertem ITO (Indium Zinn Oxid) der Dicke 50 nm beschichtet sind, bilden die Substrate für die OLEDs. Die Substrate werden nass gereinigt (Spülmaschine, Reiniger Merck Extran), anschließend 15 min lang bei 250 °C ausgeheizt und vor der Beschichtung zuerst mit einem Sauerstoffplasma und danach mit einem Argonplasma behandelt.

Die OLEDs haben prinzipiell folgenden Schichtaufbau: Substrat / Lochinjektionsschicht (HIL) / Lochtransportschicht (HTL) / Zwischenschicht (IL) / Elektronenblockerschicht (EBL) / Emissionsschicht (EML) / Lochblockierschicht (HBL) / Elektronentransportschicht (ETL) / und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs benötigten Materialien sind in Tabelle 3 gezeigt.

Alle Materialien werden in einer Vakuumkammer thermisch aufgedampft. Dabei besteht die Emissionsschicht immer aus einem Matrixmaterial (Hostmaterial, Wirtsmaterial) und der emittierenden Verbindung, die als Dotand vorliegt. Diese wird dem Matrixmaterial durch Coverdampfung in einem bestimmten Volumenanteil beigemischt. Eine Angabe wie H1:D1 (95%:5%) bedeutet hierbei, dass das Material H1 in einem Volumenanteil von 95% und D1 in einem Anteil von 5% in der Schicht vorliegt. Analog besteht die Elektronentransportschicht aus einer Mischung von zwei Materialien.

Die OLEDs werden standardmäßig charakterisiert. Hierfür werden die Elektrolumineszenzspektren, die Stromeffizienz (gemessen in cd/A), die Leistungseffizienz (gemessen in lm/W) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik, sowie die Lebensdauer bestimmt. Die Elektrolumineszenzspektren werden bei einer Leuchtdichte von 1000 cd/$m^2$ bestimmt und daraus die CIE 1931 x und y Farbkoordinaten berechnet. Die Angabe U1000 in Tabelle 2 bezeichnet die Spannung, die für eine Leuchtdichte von 1000 cd/$m^2$ benötigt wird. SE1000 und LE1000 bezeichnen die Strom- bzw. Leistungseffizienz, die bei 1000 cd/$m^2$ erreicht werden. EQE1000 schließlich bezeichnet die externe Quanteneffizienz bei einer Betriebsleuchtdichte von 1000 cd/$m^2$.

Der Roll-off wird definiert als EQE bei 5000 cd/$m^2$ geteilt durch EQE bei 500 cd/$m^2$, d.h. ein hoher Wert entspricht einem geringen Abfall der Effizienz bei hohen Leuchtdichten, was vorteilhaft ist.

Als Lebensdauer LD wird die Zeit definiert, nach der die Leuchtdichte bei Betrieb mit konstantem Strom von der Startleuchtdichte auf einen gewissen Anteil L1 absinkt. Eine Angabe von j0 = 10mA/$cm^2$, L1 = 80% in Tabelle 2 bedeutet, dass die Leuchtdichte bei Betrieb mit 10 mA/$cm^2$ nach der Zeit LD auf 80% ihres Anfangswertes absinkt.

Als emittierender Dotand in der Emissionsschicht wird die Verbindung D1 eingesetzt, die einen energetischen Abstand zwischen $S_1$ und $T_1$ von 0.09 eV aufweist.

Die Daten der verschiedenen OLEDs sind in Tabelle 2 zusammengefasst. Das Beispiel V1 ist ein Vergleichsbeispiel, das Verbindung CBP gemäß dem Stand der Technik als Matrixmaterial enthält. Die Beispiele E1-E7 zeigen Daten von erfindungsgemäßen OLEDs, die als Matrixmaterialien Verbindungen gemäß den Formeln (I) oder (II) enthalten.

Die gemessenen Leistungsdaten der OLEDs zeigen beispielhaft, dass mit einem Aufbau der emittierenden Schicht gemäß der vorliegenden Anmeldung hervorragende Werte für Spannung, Effizienz, Roll-Off und Lebensdauer der OLEDs erhalten werden. Die Leistungsdaten sind typischerweise besser als diejenigen, die mit einem Aufbau der emittierenden Schicht gemäß dem Stand der Technik (vgl. Uoyama et al., Nature 2012, Vol. 492, 234), bei dem CBP als Matrixmaterial der emittierenden Schicht verwendet wird, erhalten werden.

Insbesondere werden hervorragende Werte für die Betriebsspannung U1000, die externe Quanteneffizienz EQE, die Lebensdauer und den Roll-Off erhalten (Tabelle 2).

Tabelle 1: Aufbau der OLEDs

| Bsp | HIL Dicke | HTL Dicke | IL Dicke | EBL Dicke | EML Dicke | HBL Dicke | ETL Dicke |
|---|---|---|---|---|---|---|---|
| V1 | HAT 5nm | SpA1 70nm | HAT 5nm | SpMA1 20nm | CBP:D1 (95%:5%) 15nm | IC1 10nm | ST2:LiQ (50%:50%) 40nm |
| E1 | HAT 5nm | SpA1 70nm | HAT 5nm | SpMA1 20nm | H1:D1 (95%:5%) 15nm | IC1 10nm | ST2:LiQ (50%:50%) 40nm |
| E2 | HAT 5nm | SpA1 70nm | HAT 5nm | SpMA1 20nm | H2:D1 (95%:5%) 15nm | IC1 10nm | ST2:LiQ (50%:50%) 40nm |
| E3 | HAT 5nm | SpA1 70nm | HAT 5nm | SpMA1 20nm | H3:D1 (95%:5%) 15nm | IC1 10nm | ST2:LiQ (50%:50%) 40nm |
| E4 | HAT 5nm | SpA1 70nm | HAT 5nm | SpMA1 20nm | H4:D1 (95%:5%) 15nm | IC1 10nm | ST2:LiQ (50%:50%) 40nm |
| E5 | HAT 5nm | SpA1 70nm | HAT 5nm | SpMA1 20nm | H5:D1 (95%:5%) 15nm | IC1 10nm | ST2:LiQ (50%:50%) 40nm |
| E6 | HAT 5nm | SpA1 70nm | HAT 5nm | SpMA1 20nm | H6:D1 (95%:5%) 15nm | IC1 10nm | ST2:LiQ (50%:50%) 40nm |
| E7 | HAT 5nm | SpA1 70nm | HAT 5nm | SpMA1 20nm | H7:D1 (95%:5%) 15nm | IC1 10nm | ST2:LiQ (50%:50%) 40nm |

Tabelle 2: Daten der OLEDs

| Bsp | U1000 (V) | SE1000 (cd/A) | LE1000 (lm/W) | EQE 1000 | CIE x/y bei 1000 $cd/m^2$ | Roll-Off | L0;j0 | L1 % | LD (h) | PLQE % | $\lambda_{exc}$ nm |
|---|---|---|---|---|---|---|---|---|---|---|---|
| V1 | 4.2 | 44 | 33 | 14.1% | 0.25/0.58 | 0.60 | $10mA/cm^2$ | 80 | 23 | 100 | 350 |
| E1 | 3.7 | 47 | 41 | 15.0% | 0.31/0.58 | 0.65 | $10mA/cm^2$ | 80 | 38 | 65 | 350 |
| E2 | 3.6 | 52 | 46 | 16.3% | 0.33/0.58 | 0.70 | $10mA/cm^2$ | 80 | 32 | 70 | 350 |
| E3 | 3.5 | 44 | 40 | 13.7% | 0.32/0.58 | 0.67 | $10mA/cm^2$ | 80 | 57 | 62 | 350 |
| E4 | 3.9 | 39 | 31 | 12.8% | 0.33/0.57 | 0.62 | $10mA/cm^2$ | 80 | 43 | 74 | 350 |
| E5 | 3.9 | 50 | 40 | 15.3% | 0.30/0.58 | 0.64 | $10mA/cm^2$ | 80 | 21 | 82 | 350 |
| E6 | 4.1 | 45 | 35 | 14.0% | 0.28/0.57 | 0.62 | $10mA/cm^2$ | 80 | 33 | 94 | 350 |
| E7 | 4.4 | 39 | 28 | 12.2% | 0.28/0.57 | 0.58 | $10mA/cm^2$ | 80 | 48 | 77 | 350 |

Tabelle 3: Strukturformeln der Materialien für die OLEDs

| | |
|---|---|
| 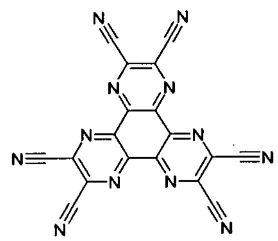 | 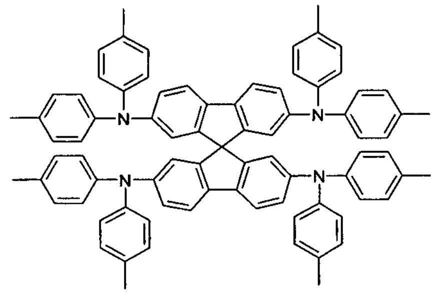 |
| HAT | SpA1 |
| 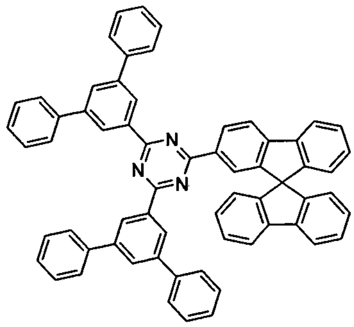 | 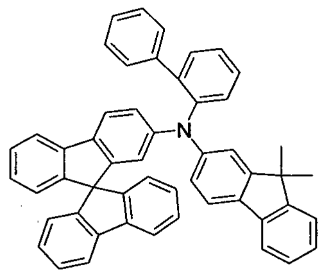 |
| ST2 | SpMA1 |
| 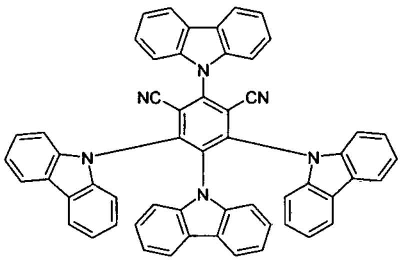 | 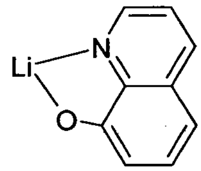 |
| D1 | LiQ |
| 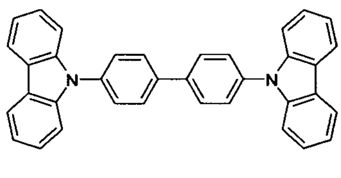 | 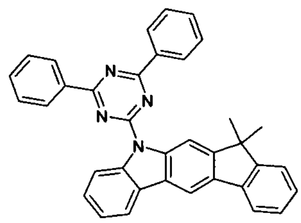 |
| CBP (Stand der Technik) | IC1 |
| 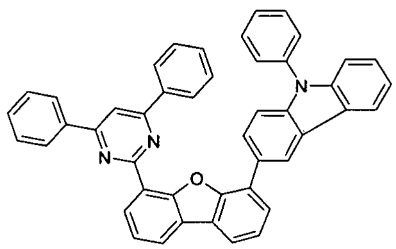 | 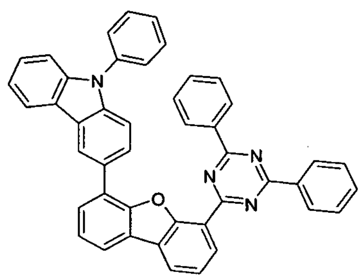 |
| H1 | H2 |

(fortgesetzt)

| | |
|---|---|
| 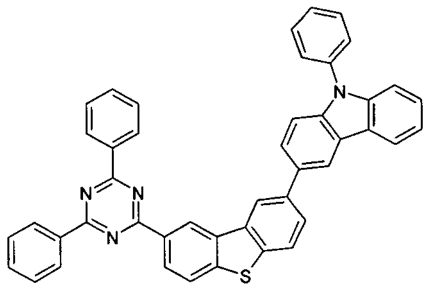 | 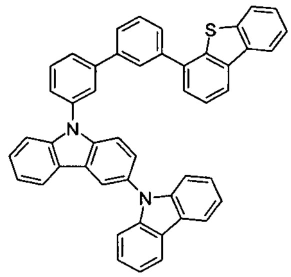 |
| H3 | H4 |
| 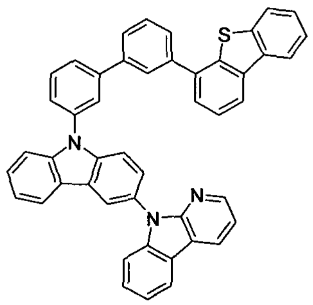 | 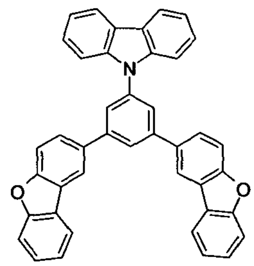 |
| H5 | H6 |
| 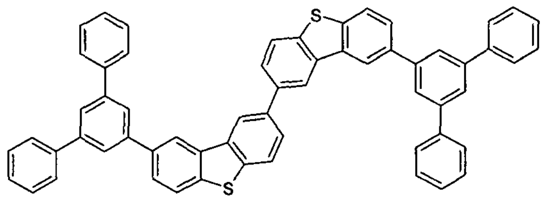 | |
| H7 | |

## Patentansprüche

1. Organische Elektrolumineszenzvorrichtung,
enthaltend eine emittierende Schicht, die als emittierende Verbindung eine Verbindung E und als Matrixmaterial eine Verbindung M enthält, wobei die Verbindung E eine betragsmäßige Differenz zwischen den Energien des $S_1$ und des $T_1$-Zustands von höchstens 0.15 eV aufweist, und
wobei die Verbindung E eine aromatische Verbindung ist, die sowohl mindestens einen Donor- als auch mindestens einen Akzeptorsubstituenten aufweist,
wobei der mindestens eine Donorsubstituent gewählt ist aus wahlweise substituierten Diarylaminogruppen, Diheteroarylaminogruppen, Carbazolgruppen und Carbazolderivaten, und
wobei der mindestens eine Akzeptorsubstituent gewählt ist aus Cyanogruppen und wahlweise substituierten elektronenarmen Heteroarylgruppen, und
wobei die Verbindung M einer Formel (I) oder (II) entspricht

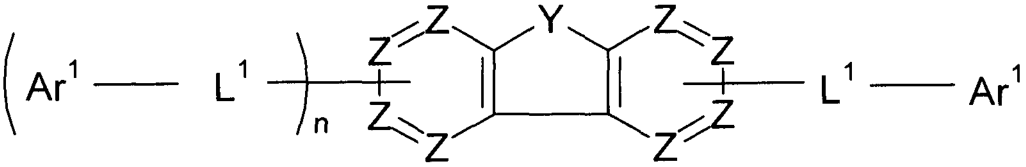

Formel (I)

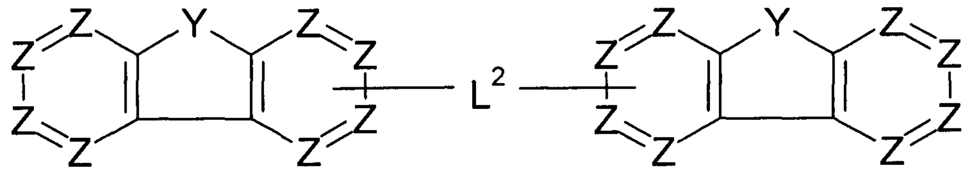

Formel (II),

für die gilt:

Y ist bei jedem Auftreten gleich oder verschieden O, S oder Se;
Z ist bei jedem Auftreten gleich oder verschieden $CR^1$, C oder N, wobei Z genau dann gleich C ist, wenn eine Gruppe $L^1$ bzw. $L^2$ zusammen mit der daran gebundenen Gruppe an Z gebunden ist;
$L^1$, $L^2$ ist bei jedem Auftreten gleich oder verschieden eine Einfachbindung oder eine divalente Gruppe;
$Ar^1$ ist bei jedem Auftreten gleich oder verschieden ein aromatisches oder heteroaromatisches Ringsystem mit 5 bis 40 aromatischen Ringatomen, das mit einem oder mehreren Resten $R^2$ substituiert sein kann;
$R^1$, $R^2$ ist bei jedem Auftreten gleich oder verschieden H, D, F, $C(=O)R^3$, CN, $Si(R^3)_3$, $N(R^3)_2$, $P(=O)(R^3)_2$, $OR^3$, $S(=O)R^3$, $S(=O)_2R^3$, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten $R^3$ substituiert sein können und wobei eine oder mehrere $CH_2$-Gruppen in den oben genannten Gruppen durch - $R^3C=CR^3$-, -C≡C-, $Si(R^3)_2$, C=O, $C=NR^3$, -C(=O)O-, $-C(=O)NR^3-$, $NR^3$, $P(=O)(R^3)$, -O-, -S-, SO oder $SO_2$ ersetzt sein können, oder ein aromatisches oder heteroaromatisches Ringsystem mit 5 bis 30 aromatischen Ringatomen, das jeweils durch einen oder mehrere Reste $R^3$ substituiert sein kann, wobei zwei oder mehr Reste $R^1$ bzw. $R^2$ miteinander verknüpft sein können und einen Ring bilden können;
$R^3$ ist bei jedem Auftreten gleich oder verschieden H, D, F, $C(=O)R^4$, CN, $Si(R^4)_3$, $N(R^4)_2$, $P(=O)(R^4)_2$, $OR^4$, $S(=O)R^4$, $S(=O)_2R^4$, eine geradkettige Alkyl- oder Alkoxygruppe mit 1 bis 20 C-Atomen oder eine verzweigte oder cyclische Alkyl- oder Alkoxygruppe mit 3 bis 20 C-Atomen oder eine Alkenyl- oder Alkinylgruppe mit 2 bis 20 C-Atomen, wobei die oben genannten Gruppen jeweils mit einem oder mehreren Resten $R^4$ substituiert sein können und wobei eine oder mehrere $CH_2$-Gruppen in den oben genannten Gruppen durch - $R^4C=CR^4$-, -C≡C-, $Si(R^4)_2$, C=O, $C=NR^4$, -C(=O)O-, $-C(=O)NR^4-$, $NR^4$, $P(=O)(R^4)$, -O-, -S-, SO oder $SO_2$ ersetzt sein können, oder ein aromatisches oder heteroaromatisches Ringsystem mit 5 bis 30 aromatischen Ringatomen, das jeweils durch einen oder mehrere Reste $R^4$ substituiert sein kann, wobei zwei oder mehr Reste $R^3$ miteinander verknüpft sein können und einen Ring bilden können;
$R^4$ ist bei jedem Auftreten gleich oder verschieden H, D, F oder ein aliphatischer, aromatischer oder heteroaromatischer organischer Rest mit 1 bis 20 C-Atomen, in dem auch ein oder mehrere H-Atome durch D oder F ersetzt sein können; dabei können zwei oder mehr Substituenten $R^4$ miteinander verknüpft sein und einen Ring bilden;
n ist gleich 0 oder 1,

wobei die Energien durch quantenchemische Rechnung bestimmt werden, wie in den Ausführungsbeispielen beschrieben.

2. Organische Elektrolumineszenzvorrichtung nach Anspruch 1, **dadurch gekennzeichnet, dass** in Formel (I) und (II) nicht mehr als eine Gruppe Z pro Ring gleich N ist.

3. Organische Elektrolumineszenzvorrichtung nach Anspruch 1 oder 2, **dadurch gekennzeichnet, dass** in Formel (I) und (II) die Gruppe Y bei jedem Auftreten gleich oder verschieden O oder S ist.

4. Organische Elektrolumineszenzvorrichtung nach einem oder mehreren der Ansprüche 1 bis 3, **dadurch gekennzeichnet, dass** in Formel (I) die Gruppe $L^1$ bei jedem Auftreten gleich oder verschieden gewählt ist aus einer Einfachbindung, $Si(R^2)_2$ oder einem aromatischen oder heteroaromatischen Ringsystem mit 5 bis 30 aromatischen Ringatomen, das mit einem oder mehreren Resten $R^2$ substituiert sein kann.

5. Organische Elektrolumineszenzvorrichtung nach einem oder mehreren der Ansprüche 1 bis 4, **dadurch gekennzeichnet, dass** in Formel (II) die Gruppe $L^2$ gewählt ist aus einer Einfachbindung oder einem aromatischen oder heteroaromatischen Ringsystem mit 5 bis 60 aromatischen Ringatomen, das mit einem oder mehreren Resten $R^2$

substituiert sein kann.

6. Organische Elektrolumineszenzvorrichtung nach einem oder mehreren der Ansprüche 1 bis 5, **dadurch gekennzeichnet, dass** in Formel (I) die Gruppe $Ar^1$ gewählt ist aus wahlweise mit Resten $R^2$ substituiertem Phenyl, Biphenyl, Terphenyl, Quaterphenyl, Fluorenyl, Spirobifluorenyl, Indenofluorenyl, Naphthyl, Anthracenyl, Phenanthrenyl, Pyrenyl, Fluoranthenyl, Furanyl, Benzofuranyl, Isobenzofuranyl, Dibenzofuranyl, Thiophenyl, Benzothiophenyl, Isobenzothiophenyl, Dibenzothiophenyl, Pyrrolyl, Indolyl, Isoindolyl, Carbazolyl, Indolocarbazolyl, Indenocarbazolyl, Pyridyl, Chinolinyl, Isochinolinyl, Acridyl, Pyrazolyl, Imidazolyl, Benzimidazolyl, Pyridazyl, Pyrimidyl, Pyrazinyl und Phenanthrolyl.

7. Organische Elektrolumineszenzvorrichtung nach einem oder mehreren der Ansprüche 1 bis 6, **dadurch gekennzeichnet, dass** die Formel (I) einer der Formeln (I-1) bis (I-4) enspricht

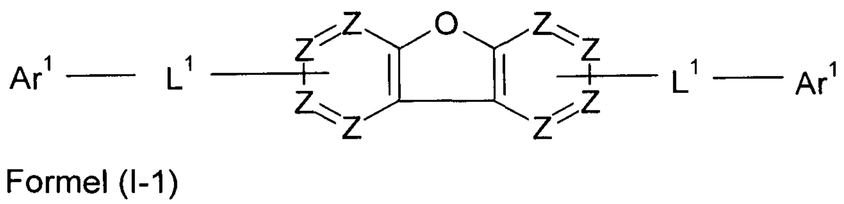

Formel (I-1)

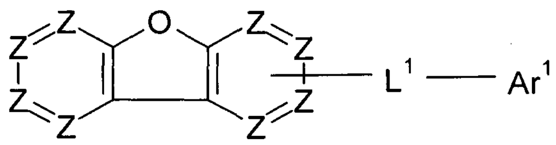

Formel (I-2)

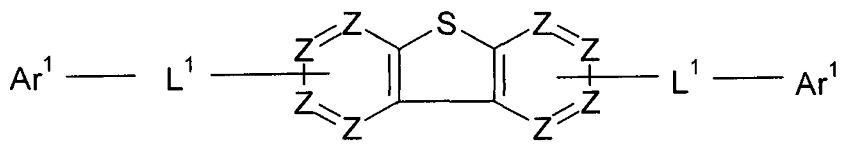

Formel (I-3)

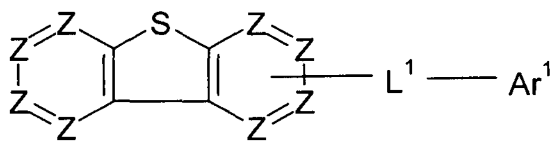

Formel (I-4),

wobei die auftretenden Gruppen definiert sind wie in einem oder mehreren der Ansprüche 1 bis 6.

8. Organische Elektrolumineszenzvorrichtung nach einem oder mehreren der Ansprüche 1 bis 7, **dadurch gekennzeichnet, dass** die Formel (II) einer der Formeln (II-1) bis (II-3) entspricht

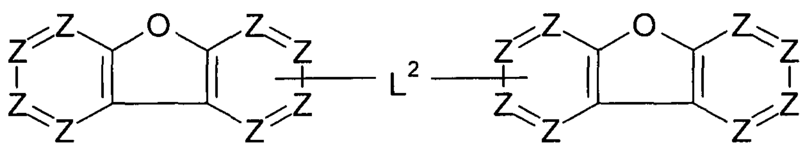

Formel (II-1)

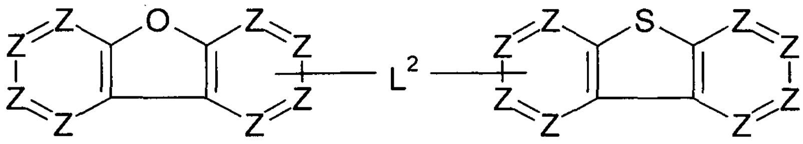

Formel (II-2)

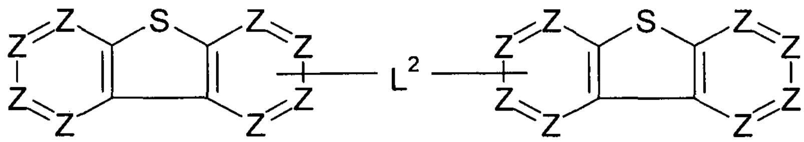

Formel (II-3),

wobei die auftretenden Gruppen definiert sind wie in einem oder mehreren der Ansprüche 1 bis 6.

**9.** Organische Elektrolumineszenzvorrichtung nach Anspruch 8, **dadurch gekennzeichnet, dass** die Gruppe $L^2$ gewählt ist aus einer Einfachbindung oder einer Einheit der Formel (L2)

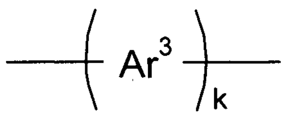

Formel (L2),

wobei gilt:

$Ar^3$ ist bei jedem Auftreten gleich oder verschieden eine Arylen- oder Heteroarylengruppe mit 6 bis 14 aromatischen Ringatomen, die mit einem oder mehreren Resten $R^2$ substituiert sein kann; und
k ist gleich 1, 2, 3 oder 4.

**10.** Organische Elektrolumineszenzvorrichtung nach einem oder mehreren der Ansprüche 1 bis 9, **dadurch gekennzeichnet, dass** die emittierende Schicht im Wesentlichen aus der Verbindung M der Formel (I) oder (II) und der Verbindung E besteht.

**11.** Organische Elektrolumineszenzvorrichtung nach einem oder mehreren der Ansprüche 1 bis 10, **dadurch gekennzeichnet, dass** der Anteil der Verbindung M zwischen 80 % und 99 % liegt, und der Anteil der Verbindung E zwischen 1 und 20 % liegt.

**12.** Organische Elektrolumineszenzvorrichtung nach einem oder mehreren der Ansprüche 1 bis 11, **dadurch gekennzeichnet, dass** für die Energie des $T_1$-Zustands der Verbindung M gemäß Formel (I) oder (II) ($T_1$(M)) und die Energie des $T_1$-Zustands der Verbindung E ($T_1$(E)) gilt:

$$T_1(M) \geq T_1(E)$$

wobei die Energien der $T_1$-Zustände durch quantenchemische Rechnung bestimmt werden, wie in den Ausführungsbeispielen beschrieben.

**13.** Organische Elektrolumineszenzvorrichtung nach einem oder mehreren der Ansprüche 1 bis 12, **dadurch gekennzeichnet, dass** die Verbindung E eine lumineszente Verbindung ist, die eine Lumineszenzquanteneffizienz von mindestens 60 % aufweist.

**14.** Organische Elektrolumineszenzvorrichtung nach einem oder mehreren der Ansprüche 1 bis 13, **dadurch gekennzeichnet, dass** die betragsmäßige Differenz zwischen den Energien des $S_1$- und des $T_1$-Zustands der Verbindung E kleiner oder gleich 0.10 eV ist, wobei die Energien durch quantenchemische Rechnung bestimmt werden, wie in den Ausführungsbeispielen beschrieben.

**15.** Organische Elektrolumineszenzvorrichtung nach einem oder mehreren der Ansprüche 1 bis 14, **dadurch gekennzeichnet, dass** für das LUMO der Verbindung E (LUMO(E)) und das HOMO der Verbindung M (HOMO(M)) gilt:

$$\mathrm{LUMO(E)} - \mathrm{HOMO(M)} > S_1(\mathrm{E}) - 0.4\ \mathrm{eV}$$

wobei $S_1$(E) der erste angeregte Singulettzustand $S_1$ der Verbindung E ist und die Energien LUMO(E) und HOMO(M) durch quantenchemische Rechnungen bestimmt werden, wie in den Ausführungsbeispielen beschrieben.

**16.** Verfahren zur Herstellung einer organischen Elektrolumineszenzvorrichtung nach einem oder mehreren der Ansprüche 1 bis 15, **dadurch gekennzeichnet, dass** mindestens eine Schicht mit einem Sublimationsverfahren aufgebracht wird und/oder dass mindestens eine Schicht mit einem OVPD-Verfahren oder mit Hilfe einer Trägergassublimation aufgebracht wird und/oder dass mindestens eine Schicht aus Lösung, durch Spincoating oder mit einem Druckverfahren aufgebracht wird.

## Claims

**1.** Organic electroluminescent device
comprising an emitting layer which comprises a compound E as emitting compound and a compound M as matrix material,
where compound E has a difference in value between the energies of the $S_1$ and $T_1$ states of at most 0.15 eV,
where compound E is an aromatic compound which contains both at least one donor substituent and also at least one acceptor substituent,
where the at least one donor substituent is selected from optionally substituted diarylamino groups, diheteroarylamino groups, carbazole groups and carbazole derivatives, and
where the at least one acceptor substituent is selected from cyano groups and optionally substituted electron-deficient heteroaryl groups, and
where compound M conforms to a formula (I) or (II)

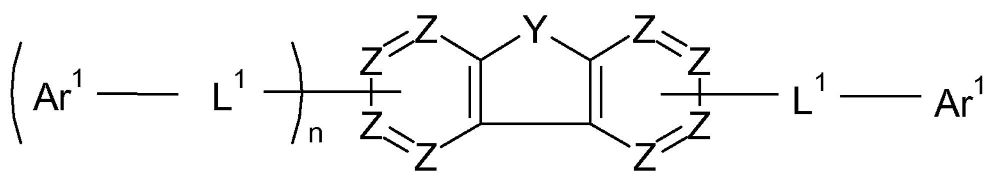

formula (I)

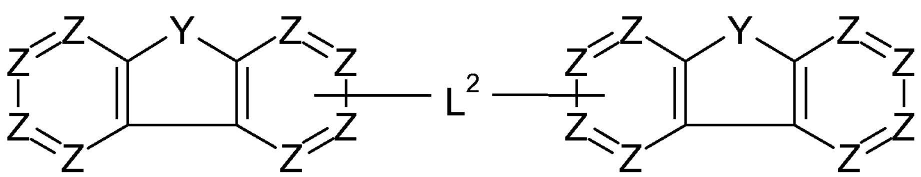

formula (II),

for which:

Y is on each occurrence, identically or differently, O, S or Se;

Z is on each occurrence, identically or differently, $CR^1$, C or N, where Z is equal to C precisely if a group $L^1$ or $L^2$ is bonded to Z together with the group bonded thereto;
$L^1$, $L^2$ are on each occurrence, identically or differently, a single bond or a divalent group;
$Ar^1$ is on each occurrence, identically or differently, an aromatic or heteroaromatic ring system having 5 to 40 aromatic ring atoms, which may be substituted by one or more radicals $R^2$;
$R^1$, $R^2$ are on each occurrence, identically or differently, H, D, F, $C(=O)R^3$, CN, $Si(R^3)_3$, $N(R^3)^2$, $P(=O)(R^3)_2$, $OR^3$, $S(=O)R^3$, $S(=O)_2R^3$, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals $R^3$ and where one or more $CH_2$ groups in the above-mentioned groups may be replaced by $-R^3C=CR^3-$, -C≡C-, $Si(R^3)_2$, C=O, $C=NR^3$, -C(=O)O-, $-C(=O)NR^{3-}$, $NR^3$, $P(=O)(R^3)$, -O-, -S-, SO or $SO_2$, or an aromatic or heteroaromatic ring system having 5 to 30 aromatic ring atoms, which may in each case be substituted by one or more radicals $R^3$, where two or more radicals $R^1$ or $R^2$ may be linked to one another and may form a ring;
$R^3$ is on each occurrence, identically or differently, H, D, F, $C(=O)R^4$, CN, $Si(R^4)_3$, $N(R^4)_2$, $P(=O)(R^4)_2$, $OR^4$, $S(=O)R^4$, $S(=O)_2R^4$, a straight-chain alkyl or alkoxy group having 1 to 20 C atoms or a branched or cyclic alkyl or alkoxy group having 3 to 20 C atoms or an alkenyl or alkynyl group having 2 to 20 C atoms, where the above-mentioned groups may in each case be substituted by one or more radicals $R^4$ and where one or more $CH_2$ groups in the above-mentioned groups may be replaced by $-R^4C=CR^4-$, -C≡C-, $Si(R^4)_2$, C=O, $C=NR^4$, -C(=O)O-, $-C(=O)NR^4-$, $NR^4$, $P(=O)(R^4)$, -O-, -S-, SO or $SO_2$, or an aromatic or heteroaromatic ring system having 5 to 30 aromatic ring atoms, which may in each case be substituted by one or more radicals $R^4$, where two or more radicals $R^3$ may be linked to one another and may form a ring;
$R^4$ is on each occurrence, identically or differently, H, D, F or an aliphatic, aromatic or heteroaromatic organic radical having 1 to 20 C atoms, in which, in addition, one or more H atoms may be replaced by D or F; two or more substituents $R^4$ here may be linked to one another and may form a ring;
n is equal to 0 or 1;

where the energies are determined by quantum-chemical calculation, as described in the working examples.

**2.** Organic electroluminescent device according to Claim 1, **characterised in that** not more than one group Z per ring in formulae (I) and (II) is equal to N.

**3.** Organic electroluminescent device according to Claim 1 or 2, **characterised in that** the group Y in formulae (I) and (II) is on each occurrence, identically or differently, O or S.

**4.** Organic electroluminescent device according to one or more of Claims 1 to 3, **characterised in that** the group $L^1$ in formula (I) is selected on each occurrence, identically or differently, from a single bond, $Si(R^2)_2$ or an aromatic or heteroaromatic ring system having 5 to 30 aromatic ring atoms, which may be substituted by one or more radicals $R^2$.

**5.** Organic electroluminescent device according to one or more of Claims 1 to 4, **characterised in that** the group $L^2$ in formula (II) is selected from a single bond or an aromatic or heteroaromatic ring system having 5 to 60 aromatic ring atoms, which may be substituted by one or more radicals $R^2$.

**6.** Organic electroluminescent device according to one or more of Claims 1 to 5, **characterised in that** the group $Ar^1$ in formula (I) is selected from phenyl, biphenyl, terphenyl, quaterphenyl, fluorenyl, spirobifluorenyl, indenofluorenyl, naphthyl, anthracenyl, phenanthrenyl, pyrenyl, fluoranthenyl, furanyl, benzofuranyl, isobenzofuranyl, dibenzofuranyl, thiophenyl, benzothiophenyl, isobenzothiophenyl, dibenzothiophenyl, pyrrolyl, indolyl, isoindolyl, carbazolyl, indolocarbazolyl, indenocarbazolyl, pyridyl, quinolinyl, isoquinolinyl, acridyl, pyrazolyl, imidazolyl, benzimidazolyl, pyridazyl, pyrimidyl, pyrazinyl and phenanthrolyl, each of which is optionally substituted by radicals $R^2$.

**7.** Organic electroluminescent device according to one or more of Claims 1 to 6, **characterised in that** the formula (I) conforms to one of the formulae (I-1) to (I-4)

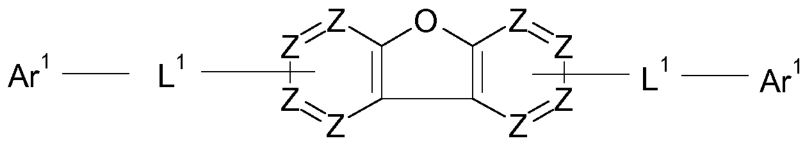

formula (I-1)

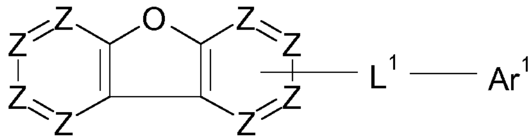

formula (I-2)

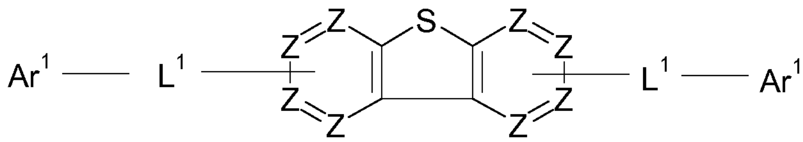

formula (I-3)

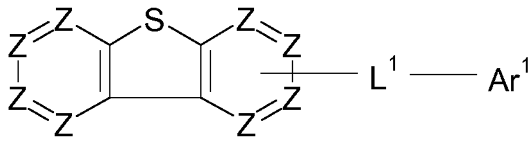

formula (I-4),

where the groups occurring are defined as in one or more of Claims 1 to 6.

**8.** Organic electroluminescent device according to one or more of Claims 1 to 7, **characterised in that** the formula (II) conforms to one of the formulae (II-1) to (II-3)

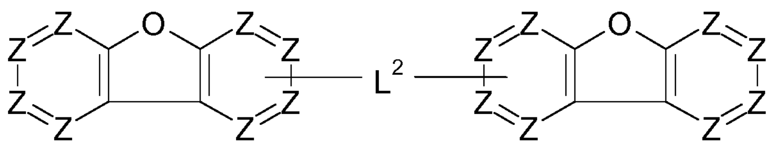

formula (II-1)

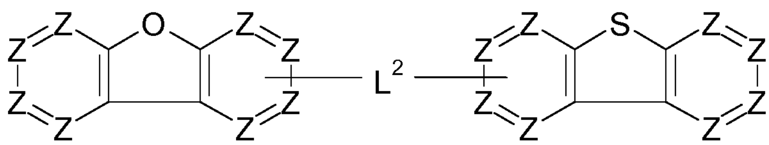

formula (II-2)

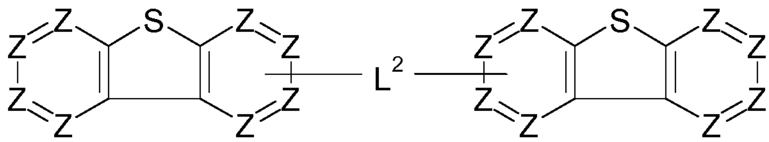

formula (II-3),

where the groups occurring are defined as in one or more of Claims 1 to 6.

**9.** Organic electroluminescent device according to Claim 8, **characterised in that** the group $L^2$ is selected from a single bond or a unit of the formula (L2)

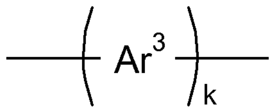

formula (L2),

where:

$Ar^3$ is on each occurrence, identically or differently, an arylene or heteroarylene group having 6 to 14 aromatic ring atoms, which may be substituted by one or more radicals $R^2$; and
k is equal to 1, 2, 3 or 4.

**10.** Organic electroluminescent device according to one or more of Claims 1 to 9, **characterised in that** the emitting layer essentially consists of compound M of the formula (I) or (II) and compound E.

**11.** Organic electroluminescent device according to one or more of Claims 1 to 10, **characterised in that** the proportion of compound M is between 80% and 99%, and the proportion of compound E is between 1 and 20%.

**12.** Organic electroluminescent device according to one or more of Claims 1 to 11, **characterised in that** the following applies to the energy of the $T_1$ state of compound M of the formula (I) or (II) ($T_1(M)$) and the energy of the $T_1$ state of compound E ($T_1(E)$):

$$T_1(M) \geq T_1(E),$$

where the energies of the $T_1$ states are determined by quantum-chemical calculation, as described in the working examples.

**13.** Organic electroluminescent device according to one or more of Claims 1 to 12, **characterised in that** compound E is a luminescent compound which has a luminescence quantum efficiency of at least 60%.

**14.** Organic electroluminescent device according to one or more of Claims 1 to 13, **characterised in that** the difference in value between the energies of the $S_1$ and $T_1$ states of compound E is less than or equal to 0.10 eV, where the energies are determined by quantum-chemical calculation, as described in the working examples.

**15.** Organic electroluminescent device according to one or more of Claims 1 to 14, **characterised in that** the following applies to the LUMO of compound E (LUMO(E)) and the HOMO of compound M (HOMO(M)):

$$LUMO(E) - HOMO(M) > S_1(E) - 0.4\ eV,$$

where $S_1(E)$ is the first excited singlet state $S_1$ of compound E, and the energies LUMO(E) and HOMO(M) are

determined by quantum-chemical calculations, as described in the working examples.

**16.** Process for the production of an organic electroluminescent device according to one or more of Claims 1 to 15, **characterised in that** at least one layer is applied by means of a sublimation process and/or **in that** at least one layer is applied by means of an OVPD process or with the aid of carrier-gas sublimation and/or **in that** at least one layer is applied from solution, by spin coating or by means of a printing process.

## Revendications

**1.** Dispositif électroluminescent organique
comprenant une couche d'émission qui comprend un composé E en tant que composé d'émission et un composé M en tant que matériau de matrice,
dans lequel le composé E présente une différence en termes de valeurs entre les énergies des états $S_1$ et $T_1$ d'au plus 0,15 eV,
dans lequel le composé E est un composé aromatique qui contient au moins un substituant donneur et également au moins un substituant accepteur,
dans lequel l'au moins un substituant donneur est sélectionné parmi les groupes diarylamino, les groupes dihétéroarylamino, les groupes carbazole et les dérivés de carbazole, en option substitués, et
dans lequel l'au moins un substituant accepteur est sélectionné parmi les groupes cyano et les groupes hétéroaryle déficients en électrons, en option substitués, et
dans lequel le composé M est conforme à une formule (I) ou (II) :

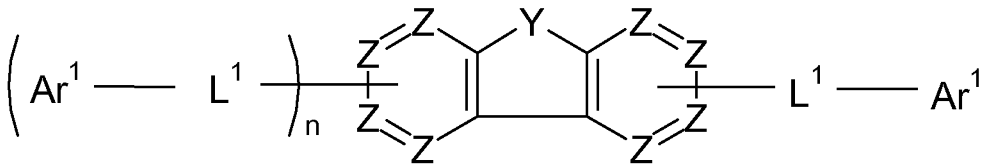

formule (I)

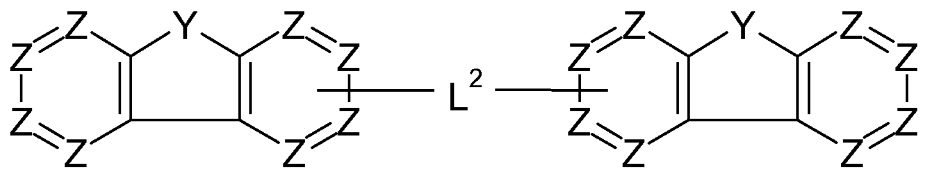

formule (II),

pour lesquelles :

Y est pour chaque occurrence, de manière identique ou différente, O, S ou Se ;
Z est pour chaque occurrence, de manière identique ou différente, $CR^1$, C ou N, dans lequel Z est égal à C précisément si un groupe $L^1$ ou $L^2$ est lié à Z en association avec le groupe qui lui est lié ;
$L^1$, $L^2$ sont pour chaque occurrence, de manière identique ou différente, une liaison simple ou un groupe divalent ;
$Ar^1$ est pour chaque occurrence, de manière identique ou différente, un système de cycle aromatique ou hétéroaromatique qui comporte 5 à 40 atomes de cycle aromatique, lequel système de cycle peut être substitué par un radical ou par plusieurs radicaux $R^2$;
$R^1$, $R^2$ sont pour chaque occurrence, de manière identique ou différente, H, D, F, $C(=O)R^3$, CN, $Si(R^3)_3$, $N(R^3)_2$, $P(=O)(R^3)_2$, $OR^3$, $S(=O)R^3$ $S(=O)_2R^3$, un groupe alkyle ou alcoxy en chaîne droite qui comporte 1 à 20 atome(s) de C ou un groupe alkyle ou alcoxy ramifié ou cyclique qui comporte 3 à 20 atomes de C ou un groupe alkényle ou alkynyle qui comporte 2 à 20 atomes de C, dans lequel les groupes qui ont été mentionnés ci-avant peuvent dans chaque cas être substitués par un radical ou par plusieurs radicaux $R^3$ et dans lequel un ou plusieurs groupe(s) $CH_2$ dans les groupes qui ont été mentionnés ci-avant peut/peuvent être remplacé(s) par $-R^3C=CR^3-$, $-C\equiv C-$, $Si(R^3)_2$, C=O, $C=NR^3$, -C(=O)O-, $-C(=O)NR^3-$, $NR^3$, $P(=O)(R^3)$, -O-, -S-, SO ou $SO_2$, ou un système de

cycle aromatique ou hétéroaromatique qui comporte 5 à 30 atomes de cycle aromatique, lequel système de cycle peut dans chaque cas être substitué par un radical ou par plusieurs radicaux $R^3$, dans lequel deux radicaux $R^1$ ou $R^2$ ou plus peuvent être liés l'un à l'autre ou les uns aux autres et peuvent former un cycle ;

$R^3$ est pour chaque occurrence, de manière identique ou différente, H, D, F, $C(=O)R^4$, CN, $Si(R^4)_3$, $N(R^4)_2$, $P(=O)(R^4)_2$, $OR^4$, $S(=O)R^4$, $S(=O)_2R^4$, un groupe alkyle ou alcoxy en chaîne droite qui comporte 1 à 20 atome(s) de C ou un groupe alkyle ou alcoxy ramifié ou cyclique qui comporte 3 à 20 atomes de C ou un groupe alkényle ou alkynyle qui comporte 2 à 20 atomes de C, dans lequel les groupes qui ont été mentionnés ci-avant peuvent dans chaque cas être substitués par un radical ou par plusieurs radicaux $R^4$ et dans lequel un ou plusieurs groupe(s) $CH_2$ dans les groupes qui ont été mentionnés ci-avant peut/peuvent être remplacé(s) par $-R^4C=CR^4-$, -C≡C-, $Si(R^4)_2$, C=O, $C=NR^4$, -C(=O)O-, $-C(=O)NR^4-$, $NR^4$, $P(=O)(R^4)$, -O-, -S-, SO ou $SO_2$, ou un système de cycle aromatique ou hétéroaromatique qui comporte 5 à 30 atomes de cycle aromatique, lequel système de cycle peut dans chaque cas être substitué par un radical ou par plusieurs radicaux $R^4$, dans lequel deux radicaux $R^3$ ou plus peuvent être liés l'un à l'autre ou les uns aux autres et peuvent former un cycle ;

$R^4$ est pour chaque occurrence, de manière identique ou différente, H, D, F ou un radical organique aliphatique, aromatique ou hétéroaromatique qui comporte 1 à 20 atome(s) de C, dans lequel, en outre, un ou plusieurs atome(s) de H peut/ peuvent être remplacé(s) par D ou F ; deux substituants $R^4$ ou plus peuvent ici être liés l'un à l'autre ou les uns aux autres et peuvent former un cycle ;

n est égal à 0 ou 1 ;

dans lequel les énergies sont déterminées au moyen d'un calcul de la chimie quantique, comme décrit selon les exemples concrets.

2. Dispositif électroluminescent organique selon la revendication 1, **caractérisé en ce que** pas plus d'un groupe Z par cycle dans les formules (I) et (II) n'est égal à N.

3. Dispositif électroluminescent organique selon la revendication 1 ou 2, **caractérisé en ce que** le groupe Y dans les formules (I) et (II) est, pour chaque occurrence, de manière identique ou différente, O ou S.

4. Dispositif électroluminescent organique selon une ou plusieurs des revendications 1 à 3, **caractérisé en ce que** le groupe $L^1$ dans la formule (I) est sélectionné pour chaque occurrence, de manière identique ou différente, parmi une liaison simple, $Si(R^2)_2$ ou un système de cycle aromatique ou hétéroaromatique qui comporte 5 à 30 atomes de cycle aromatique, lequel système de cycle peut être substitué par un radical ou par plusieurs radicaux $R^2$.

5. Dispositif électroluminescent organique selon une ou plusieurs des revendications 1 à 4, **caractérisé en ce que** le groupe $L^2$ dans la formule (II) est sélectionné parmi une liaison simple ou un système de cycle aromatique ou hétéroaromatique qui comporte 5 à 60 atomes de cycle aromatique, lequel système de cycle peut être substitué par un radical ou par plusieurs radicaux $R^2$.

6. Dispositif électroluminescent organique selon une ou plusieurs des revendications 1 à 5, **caractérisé en ce que** le groupe $Ar^1$ dans la formule (I) est sélectionné parmi phényle, biphényle, terphényle, quaterphényle, fluorényle, spirobifluorényle, indénofluorényle, naphtyle, anthracényle, phénanthrényle, pyrényle, fluoranthényle, furanyle, benzofuranyle, isobenzofuranyle, dibenzofuranyle, thiophényle, benzothiophényle, isobenzothiophényle, dibenzothiophényle, pyrrolyle, indolyle, isoindolyle, carbazolyle, indolocarbazolyle, indénocarbazolyle, pyridyle, quinolinyle, isoquinolinyle, acridyle, pyrazolyle, imidazolyle, benzimidazolyle, pyridazyle, pyrimidyle, pyrazinyle et phénanthrolyle, dont chacun est en option substitué par des radicaux $R^2$.

7. Dispositif électroluminescent organique selon une ou plusieurs des revendications 1 à 6, **caractérisé en ce que** la formule (I) est conforme à l'une des formules (I-1) à (I-4) :

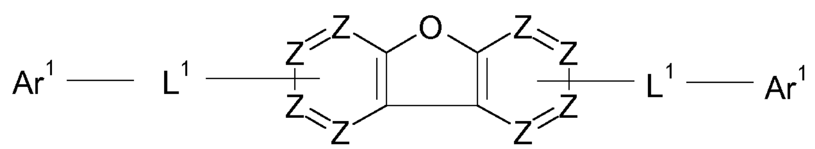

formule (I-1)

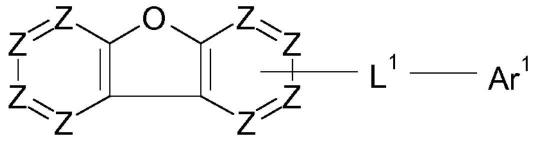

formule (I-2)

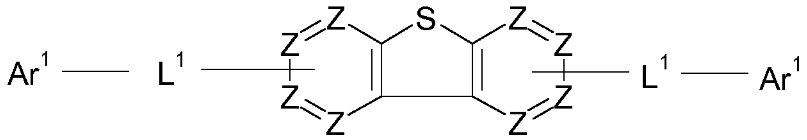

formule (I-3)

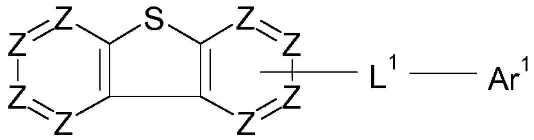

formule (I-4),

dans lesquelles les groupes qui sont présents sont définis tel que selon une ou plusieurs des revendications 1 à 6.

**8.** Dispositif électroluminescent organique selon une ou plusieurs des revendications 1 à 7, **caractérisé en ce que** la formule (I) est conforme à l'une des formules (II-1) à (II-3) :

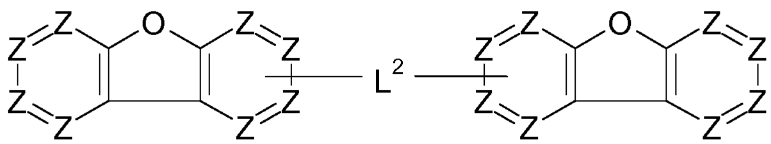

formule (II-1)

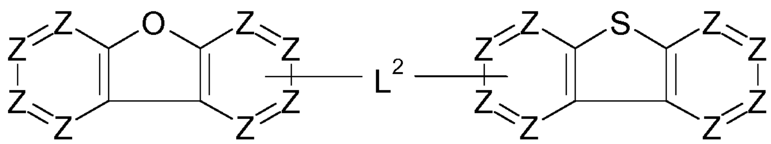

formule (II-2)

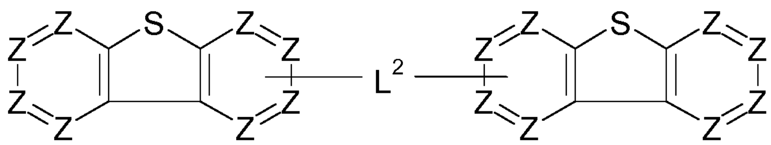

formule (II-3),

dans lesquelles les groupes qui sont présents sont définis tel que selon une ou plusieurs des revendications 1 à 6.

**9.** Dispositif électroluminescent organique selon la revendication 8, **caractérisé en ce que** le groupe $L^2$ est sélectionné parmi une liaison simple ou une unité de la formule (L2) :

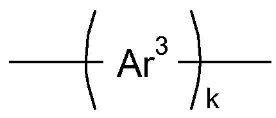

formule (L2),

dans laquelle :

$Ar^3$ est pour chaque occurrence, de manière identique ou différente, un groupe arylène ou hétéroarylène qui comporte 6 à 14 atomes de cycle aromatique, lequel groupe peut être substitué par un radical ou par plusieurs radicaux $R^2$ ; et
k est égal à 1, 2, 3 ou 4.

**10.** Dispositif électroluminescent organique selon une ou plusieurs des revendications 1 à 9, **caractérisé en ce que** la couche d'émission est essentiellement constituée par le composé M de la formule (I) ou (II) et par le composé E.

**11.** Dispositif électroluminescent organique selon une ou plusieurs des revendications 1 à 10, **caractérisé en ce que** la proportion du composé M est entre 80 % et 99 %, et la proportion du composé E est entre 1 et 20 %.

**12.** Dispositif électroluminescent organique selon une ou plusieurs des revendications 1 à 11, **caractérisé en ce que** ce qui suit s'applique à l'énergie de l'état $T_1$ du composé M de la formule (I) ou (II) ($T_1$(M)) et à l'énergie de l'état $T_1$ du composé E ($T_1$(E)) :

$$T_1(M) \geq T_1(E),$$

dans lequel les énergies des états $T_1$ sont déterminées au moyen d'un calcul de la chimie quantique, comme décrit selon les exemples concrets.

**13.** Dispositif électroluminescent organique selon une ou plusieurs des revendications 1 à 12, **caractérisé en ce que** le composé E est un composé luminescent qui présente un rendement quantique de luminescence d'au moins 60 %.

**14.** Dispositif électroluminescent organique selon une ou plusieurs des revendications 1 à 13, **caractérisé en ce que** la différence en termes de valeurs entre les énergies des états $S_1$ et $T_1$ du composé E est inférieure ou égale à 0,10 eV, dans lequel les énergies sont déterminées au moyen d'un calcul de la chimie quantique, comme décrit selon les exemples concrets.

**15.** Dispositif électroluminescent organique selon une ou plusieurs des revendications 1 à 14, **caractérisé en ce que** ce qui suit s'applique au LUMO du composé E (LUMO(E)) et au HOMO du composé M (HOMO(M)) :

$$LUMO(E) - HOMO(M) > S_1(E) - 0{,}4\ eV,$$

dans lequel $S_1$(E) est le premier état singulet excité $S_1$ du composé E, et les énergies LUMO(E) et HOMO(M) sont déterminées au moyen d'un calcul de la chimie quantique, comme décrit selon les exemples concrets.

**16.** Procédé pour la production d'un dispositif électroluminescent organique selon une ou plusieurs des revendications 1 à 15, **caractérisé en ce qu'**au moins une couche est appliquée au moyen d'un procédé de sublimation et/ou **en ce qu'**au moins une couche est appliquée au moyen d'un procédé OVPD (dépôt physique en phase vapeur organique)ou à l'aide d'une sublimation par gaz porteur et/ou **en ce qu'**au moins une couche est appliquée à partir d'une solution, au moyen d'un dépôt/revêtement à la tournette/par centrifugation ou au moyen d'un procédé d'impression.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4539507 A
- US 5151629 A
- EP 0676461 A
- WO 9827136 A
- US 2009030202 A
- US 2011260138 A
- US 2012259169 A
- WO 2012145173 A
- WO 2012048266 A
- WO 2011137072 A
- EP 2551932 A
- US 20090030202 A
- WO 2011057706 A
- WO 2011073149 A
- EP 1968131 A
- EP 2276085 A
- EP 2213662 A
- EP 1722602 A
- EP 2045848 A
- DE 102007031220
- US 8044390 B
- US 8057712 B
- WO 2009003455 A
- WO 2010094378 A
- WO 2011120709 A
- US 20100096600 A
- WO 201212145173 A


### In der Beschreibung aufgeführte Nicht-Patentliteratur


- **H. UOYAMA et al.** *Nature,* 2012, vol. 492, 234
- **G. MEHES.** *Angew. Chem. Int. Ed.,* 2012, vol. 51, 11311-11315
- *Chem. Rev.,* 2007, vol. 107, 1233
- **M. S. ARNOLD et al.** *Appl. Phys. Lett.,* 2008, vol. 92, 053301
- **UOYAMA et al.** *Nature,* 2012, vol. 492, 234